(19) **Europäisches Patentamt / European Patent Office / Office européen des brevets**

(11) **EP 4 489 753 B1**

(12) # EUROPEAN PATENT SPECIFICATION

(45) Date of publication and mention
of the grant of the patent:
**05.08.2026 Bulletin 2026/32**

(21) Application number: **23714042.1**

(22) Date of filing: **06.03.2023**

(51) International Patent Classification (IPC):
**A61K 31/436** (2006.01)   **A61L 29/08** (2006.01)
**A61L 29/16** (2006.01)   **A61L 31/10** (2006.01)
**A61L 31/16** (2006.01)

(52) Cooperative Patent Classification (CPC):
**A61K 9/1694; A61K 9/1647; A61K 31/436;
A61L 29/085; A61L 29/16; A61L 31/10;
A61L 31/16;** A61K 9/0019; A61K 9/19;
A61L 2300/416

(86) International application number:
**PCT/US2023/063792**

(87) International publication number:
**WO 2023/172876 (14.09.2023 Gazette 2023/37)**

(54) **SIROLIMUS MICROSPHERES AND METHOD OF MAKING SIROLIMUS MICROSPHERES**

SIROLIMUS-MIKROKUGELN UND VERFAHREN ZUR HERSTELLUNG VON
SIROLIMUS-MIKROKUGELN

MICROSPHÈRES DE SIROLIMUS ET PROCÉDÉ DE FABRICATION DE MICROSPHÈRES DE
SIROLIMUS

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **07.03.2022 US 202217688682**

(43) Date of publication of application:
**15.01.2025 Bulletin 2025/03**

(73) Proprietor: **M.A. Med Alliance SA
1260 Nyon (CH)**

(72) Inventors:
  • **DEYDIER, Tymele**
    **1260 Nyon (CH)**
  • **ZHANG, Zilin**
    **1260 Nyon (CH)**
  • **EKANEM, Ekanem**
    **1260 Nyon (CH)**
  • **BOLOGNESI, Guido**
    **1260 Nyon (CH)**
  • **VLADISAVLJEVIC, Goran, T.**
    **1260 Nyon (CH)**

(74) Representative: **Lavoix
Bayerstraße 83
80335 München (DE)**

(56) References cited:
  • **VLADISAVLJEVIC GORAN T. ET AL: "Long-term
    stability of droplet production by microchannel
    (step) emulsification in microfluidic silicon chips
    with large number of terraced microchannels",
    CHEMICAL ENGENEERING JOURNAL, vol. 333,
    1 February 2018 (2018-02-01), AMSTERDAM, NL,
    pages 380 - 391, XP93054086, ISSN: 1385-8947,
    DOI: 10.1016/j.cej.2017.09.141**
  • **ZHU CHENGCHENG ET AL: "Microfluidic
    preparation of PLGA microspheres as cell
    carriers with sustainable Rapa release",
    JOURNAL OF BIOMATERIALS SCIENCE.
    POLYMER EDITION., vol. 30, no. 9, 19 April 2019
    (2019-04-19), NL, pages 737 - 755, XP055841999,
    ISSN: 0920-5063, Retrieved from the Internet
    <URL:https://www.tandfonline.com/doi/pdf/
    10.1080/09205063.2019.1602930?
    needAccess=true%20rapamycin%20PLGA%
    20microparticles%20with%20cavities%20on%
    20surface> DOI: 10.1080/09205063.2019.1602930**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **VLADISAVLJEVIC GORAN T. ET AL: "Long-term stability of droplet production by microchannel (step) emulsification in microfluidic silicon chips with large number of terraced microchannels", CHEMICAL ENGENEERING JOURNAL, vol. 333, 1 February 2018 (2018-02-01), AMSTERDAM, NL, pages 380 - 391, XP093054086, ISSN: 1385-8947, DOI: 10.1016/j.cej.2017.09.141**

**Description**

BACKGROUND

Field

This disclosure is related to the field of microspheres generated by step microfluidic emulsification.

Description of the Related Art

An implantable medical device is any instrument used for diagnostic and/or therapeutic purposes such as a stent, temporary or permanent dwelling catheter or balloon, which is introduced into the human body by clinical intervention and intended to establish short term contact with living tissues, or to remain in place for an extended period. Although these devices are subject to rigorous health and safety requirements, their intimate and prolonged contact with the human body can increase the risks of adverse events and reactions, such as the formation of bacterial biofilm and restenosis. Blood vessels frequently suffer from the recurrence of restenosis following angioplasty, an adverse result of the procedure, which can be recognized as the re-narrowing of a body conduit, for example an artery previously treated for blockage. Restenosis represents one of the significant risk factors limiting the long-term success of percutaneous coronary interventions.

Angioplasty balloons and coronary stents can be coated with an anti-restenosis drug, which is preferably slowly released after contact between the device and the artery wall as part of an angioplasty procedure to inhibit neointimal hyperplasia, the main cause of restenosis. Paclitaxel and sirolimus (rapamycin) and their derivatives are currently the two major anti-restenotic drugs used in or coated on implantable, drug-eluting medical devices to limit restenosis, Paclitaxel diffuses more readily through the plaque and the artery wall, but it is less effective in suppressing restenosis and has a narrower therapeutic window as compared to sirolimus (SRL). As a result, a growing number of sirolimus-eluting stents and sirolimus coated angioplasty balloons are being developed.

A development in drug-eluting technology is the adoption of drug carrier (excipient) which is mixed with the drug to form a coating which acts as a transfer medium, facilitating drug transfer to the vessel wall. Without the excipient, the drug may form crystalline lumps on the device surface, which encourages wash off and inhibits drug transfer and absorption, especially in drug-coated balloons due to short contact time between the inflated balloon and the artery wall (30-120 s). Benefits of encapsulating drugs in carrier materials such as lipid excipients and/or biodegradable polymers include enhanced adhesion of the drug to the device surface, increased stability of the coating during handling, improved adherence to the vessel wall upon balloon expansion, and extended drug release to prolong therapeutic effects and minimize side effects such as dose-mediated toxicity. Coating for drug-eluting medical devices often consists of an excipient layer, carrying at least one therapeutic drug substance. This approach provides high initial drug levels, but the drug concentration rapidly decreases because the drug diffuses quickly out of the surface region of the coating and the coating quickly becomes depleted of drug after reaching the vessel wall. One way of achieving a more uniform drug release over prolonged time is to prepare microparticles (sometimes shaped in the form of spheres) which are composed of a drug and carrier mixture. These "micro-reservoirs" may be dispersed in a thin layer of carrier material to form the drug-release coating. After implantation, drug molecules are slowly released from the micro-reservoirs which have been deposited in the vessel wall by balloon expansion, each of which acts as miniaturized drug delivery system, releasing drug into the secondary carrier layer. The drug is then diffused through the secondary carrier material and into the living cells in the artery wall or other tissue to be treated. The secondary carrier material should initially be resistant to wash-off from the balloon or catheter surface during advancement of the catheter to the treatment site, and then have an ability to be released and transferred to adhere well to the endothelial lining of the arteries, carrying the micro-reservoirs along with it, thus providing a good geographic dispersion of micro-reservoirs over treated tissues.

Poly(lactic-co-glycolic acid) (PLGA) is the most widely used biomaterial for drug microencapsulation, generally recognized as safe by FDA and the European Medicines Agency (EMA). Currently, there are 19 FDA-approved PLGA-based drug products on the market, mainly in the form of micro-spheres. Particle size is a key factor in their design, as it affects drug encapsulation efficiency, product injectability, syringe-ability, distribution in the body, and drug release rate. Conventional methods of producing PLGA microspheres, such as spray drying and emulsification using high-speed mixers and high-pressure homogenizers followed by solvent evaporation, do not allow for a precise control of the mean particle size and show wide particle size distributions with a coefficient of variation (CV) of about 30-50%. The particle size uniformity can be improved through expensive classification processes, but these processes are associated with high drug and polymer losses. Better control over particle size can be achieved using membrane emulsification, but the CVs are still relatively high, between about 7% and about 20%.

Thus there is a continuing need for a process of manufacturing drug loaded particles that have the important properties of 1-10 micron diameter, uniform size and drug content, and inherent resistance to agglomeration for use in injectable and device coating applications. Agglomeration can be problematic as suspended particles tend to adhere one to the other creating bigger and heavier aggregates.

Recent advances in microengineering and semiconductor technologies have enabled fabrication of microfluidic chips for obtaining monodisperse droplets with CVs less than about 3%. Droplet microfluidics offer many advantages over conventional emulsification methods including unprecedented control over droplet size with no polymer/drug losses, high drug encapsulation efficiency because droplets are formed at negligible shear, under continuous processing conditions, and operation can be in closed environments enabling sterile manufacturing to meet CGMP regulations.

The main problem of conventional microfluidic devices such as single T-junctions and ψ-junctions is a low production rate because droplets are formed one at a time. Furthermore, the maximum droplet throughput is restricted by dripping to jetting transition, which must be avoided. Scaling up T-junctions and flow focusing nozzles is challenging because droplet formation in these geometries is controlled by shear rate at the dispersed/continuous phase interface, which is sensitive to fluid flow rates. As a result, small flow rate fluctuations can result in large droplet size variations, making parallelization of these devices difficult. Step microfluidic emulsification is an alternative approach of generating uniform droplets in a low shear environment by exploiting a sudden change in channel geometry from shallow channels to a deep and wide reservoir. Although the mechanism was discovered in the mid-1990s, it was largely ignored until recent years. The droplet size in step emulsification devices can be tuned solely by the internal channel geometry. The effect of fluid flow rates is negligible, which allows easy scale-up of individual microchannels. ZHU CHENGCHENG ET AL: "Microfluidic preparation of PLGA microspheres as cell carriers with sustainable Rapa release", JOURNAL OF BIOMATERIALS SCIENCE. POLYMER EDITION., vol. 30, no. 9, 19 April 2019, pages 737-755 discloses the microfluidic preparation of homogeneous and micro-disperse PLGA microspheres with sustainable sirolimus release. A capillary-based two-phase microfluidic device was designed to prepare monodisperse PLGA microspheres to load sirolimus.

## SUMMARY

The scope of protection is defined by the claims.

With prior art methods, it has been difficult to formulate microspheres in the range of below 20 microns in diameter with a CV in the range of less than about 3%. We disclose herein a method for creating microreservoirs of small size (e.g., < 20 microns), and uniform composition. The amorphous composition of the small reservoirs as described herein makes them highly useful for the volume manufacturing of drug delivery systems with precise pharmacokinetics which are capable of being delivered and applied to micro-sized features, fissures, and tissue beds in living bodies using small-sized, less traumatic device implants such as injectable slurries, stents and catheters. The microreservoirs presently disclosed may be useful for treating restenosis during or after angioplasty, for injecting into the blood supply feeding tumors, for injecting to reduce thrombosis in major arteries supplying major organs (e.g., the brain, pancreas, liver, heart), for delivering in aerosols to treat conditions in the air carrying structures in the lungs, for delivering in ointments for treatment of skin conditions, for delivering in injections and surface medications to treat degenerative eye conditions, and for delivering in oral medications, or for supplying a continuous flux of drug in a gastrointestinal tract. In some embodiments, the disclosed microreservoirs may be used to provide an oral medication containing particles which is intended to reduce systemic inflammation throughout the body, which can lead to greater longevity.

In some embodiments, disclosed is a method of generating agglomeration-resistant monodispersed and homogenous sirolimus microspheres. In some embodiments, the method comprises producing droplets comprising sirolimus (sirolimus droplets) using microfluidic step emulsification, wherein the drug loading is about 100%. In some embodiments, the method includes washing the droplets to remove poly (vinyl alcohol). In some embodiments, the method includes removing solvent from the sirolimus droplets, to form particles that are reduced in size. For example, in some embodiments, the particles are reduced in size by the volume percentage of the solvent in the microsphere.

In some embodiments, disclosed is a method of generating agglomeration-resistant monodispersed and homogenous sirolimus microspheres. In some embodiments, the method comprises washing the microspheres to remove poly (vinyl alcohol). The method comprises freeze-drying the sirolimus microspheres after solvent evaporation. In some embodiments, freeze-drying the sirolimus particles is configured to reduce particle size by about 10% to about 15%.

In some embodiments, the microfluidic chip used in the microfluidic emulsification includes a plurality of junctions. In some examples, the plurality of junctions of the microfluidic chip provide for 3D flow focusing. In some embodiments, the microfluidic chip includes any number of junctions up to ten. In some embodiments, the microfluidic chip includes seven junctions. In some embodiments, the microfluidic chip comprises 10 parallel arrays of terraced micro-grooves, wherein the microgrooves have a depth of between about 2-about 5 μm. In some examples, the plurality of parallel arrays in the microfluidic chip provide step emulsification.

In some embodiments, a surface of the microfluidic chip is washed with dichloromethane (DCM). In some embodiments, the microspheres are washed between 6-7 times. In some embodiments, the microspheres are washed at least 8 times. In some embodiments, the droplets are formed using a dripping regime. In some examples, the dripping regime is used as it can produce highly uniform droplets. In some embodiments, the microspheres can also be formed using a squeezing regime. In some embodiments, washing the microspheres further comprises centrifuging a suspension at 3500 rpm for about 10 minutes. In some embodiments, washing the microspheres further comprises adding an equal volume of about 0.05 wt% Tween 20 aqueous solution, and vortexing for 5-10 s.

In some embodiments, producing the sirolimus droplets comprise using continuous phase that does not include isopropyl acetate (IPAc). In some examples, the microspheres can be loaded with drug. Drug loading can be represented as a weight percentage (wt%), wherein the wt% is defined as the percent of drug in the solid portion of the microsphere (i.e., the combination of the drug and the polymer). The drug loading wt% of the sirolimus droplets is about 100%. The drug loading of the sirolimus microspheres is about 100 wt%. In some embodiments, removing the residual solvent from the sirolimus droplets comprises freeze-drying. In some examples, while the majority of solvent is removed by solvent evaporation, the sirolimus droplets can also be freeze-dried to remove any residual solvent. In some embodiments, the particles formed have a diameter greater than 8 $\mu$m. In some embodiments, the particles formed have a diameter between 1.8-8.4 $\mu$m. In some embodiments, the particles formed do not include Janus particles or phase separation.

BRIEF DESCRIPTION OF THE DRAWINGS

These and other features, aspects and advantages are described below with reference to the drawings, which are intended for illustrative purposes and should in no way be interpreted as limiting the scope of the embodiments. In the drawings, like reference characters denote corresponding features consistently throughout similar embodiments. The following is a brief description of each of the drawings.

Figure 1A illustrates an embodiment of a microfluidic module for step microfluidic emulsification and corresponding silicon chip.

Figure 1B illustrates a silicon chip with terraced microchannels for use in the microfluidic module of Figure 1A.

Figure 1C illustrates a magnified top view of a section of a single dead-end channel of the silicon chip of Figure 1B identified as "D-D".

Figure 1D illustrates a magnified side view of a portion of the dead-end channel of Figure 1C.

Figure 1E illustrates a terrace wall with two microchannels on a portion of the silicon chip of Figure 1B.

Figure 2 illustrates a schematic of an embodiment of a microfluidic module and Telos chip with 7 parallel 3D flow focusing junctions.

Figure 3A illustrates an embodiment of a 100 $\mu$m droplet junction chip.

Figure 3B illustrates an embodiment of a 100 $\mu$m 3D flow focusing chip.

Figure 3C illustrates an embodiment of a 30 $\mu$m 3D flow focusing chip.

Figures 4A-4D illustrate various droplet generation regimes for use in an embodiment of a microfluidic chip.

Figure 5 illustrates a graph of the Weber number of the dispersed phase ($We_{DP}$) to the continuous phase ($Ca_{cp}$) for threading, dripping, and squeezing.

Figure 6 illustrates the mean droplet diameter generated using an embodiment of a microfluidic chip shown in Figure 1B consisted of 5 $\mu$m depth channels measured over 25 hours.

Figure 7 illustrates the mean droplet diameter generated using another embodiment of a microfluidic chip shown in Figure 1B consisted of 4 $\mu$m depth channels measured over 24 hours.

Figure 8 illustrates the mean droplet diameter generated using another embodiment of a microfluidic chip shown in Figure 1B shown in Figure 1B consisted of 2 $\mu$m depth channels measured over 20 hours.

Figure 9A illustrates a SEM image of SRL-loaded PLGA particles without washing.

Figures 9B-9C illustrate SEM images of the sample of Figure 9A after 8 washes.

Figures 10A-10D and 11A-11D illustrate the effect of the continuous phase saturation with organic solvent (IPAc) on particle morphology of a sample of microspheres.

Figure 12 illustrates the effect of drug loading on the particle morphology for the particle size between 4.1 $\mu$m and 4.5 $\mu$m.

Figure 13 illustrates an SEM image of 100 % PLGA particles.

Figure 14 illustrates another graph showing the XRD patterns of pure SRL powder, pure PLGA powder, 100% SRL particles, and 100% PLGA particles.

Figure 15 shows the FTIR spectra for pure PLGA powder, pure SRL powder, and SRL-loaded PLGA particles.

Figure 16 illustrates another graph showing the FTIR patterns of 100% SRL particles, 100% PLGA particles, particles with a PLGA:SRL ratio of 4:1, and particles with a PLGA:SRL ratio of 1:4.

Figure 17 illustrates another graph showing the FTIR patterns of 100% SRL particles and particles having a PCL:SRL ratio of 4:1.

DETAILED DESCRIPTION

Disclosed are systems and methods for generating agglomeration-resistant monodispersed microspheres comprising sirolimus using step microfluidic emulsification and flow focusing microfluidic junctions. In some embodiments, the generated agglomeration-resistant monodispersed sirolimus microspheres can then be used for drug-coated implantable devices and/or subcutaneous injection. The microspheres are substantially pure sirolimus.

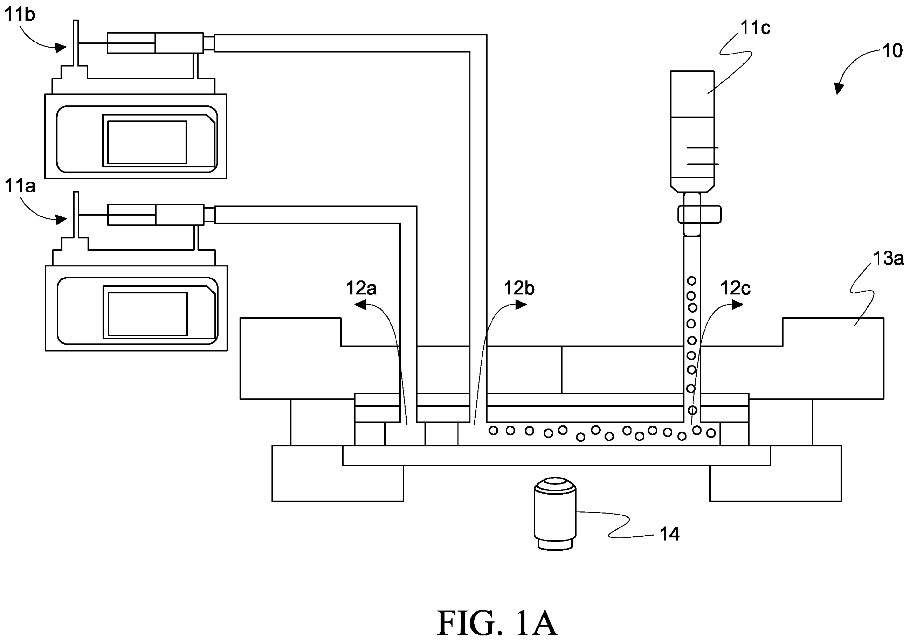

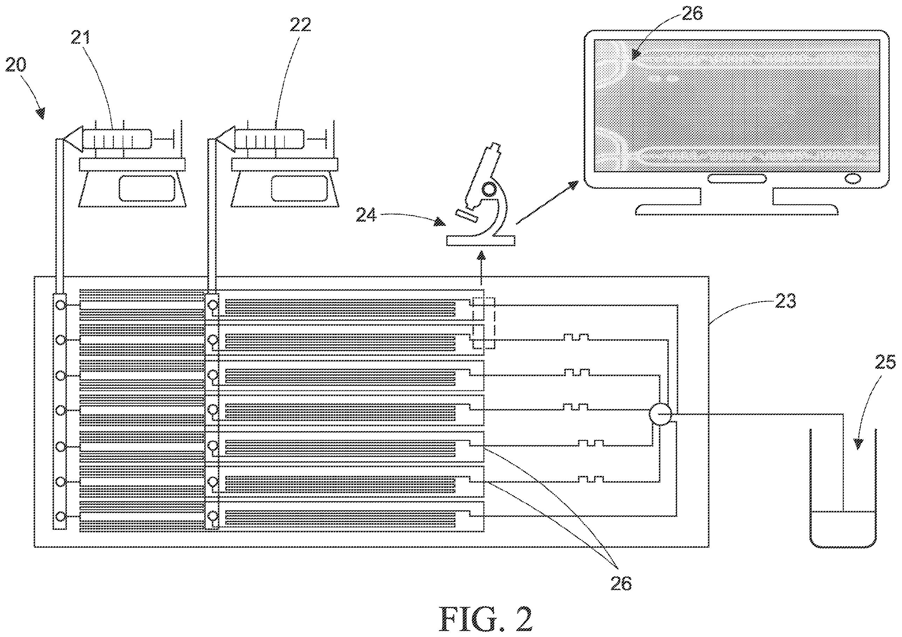

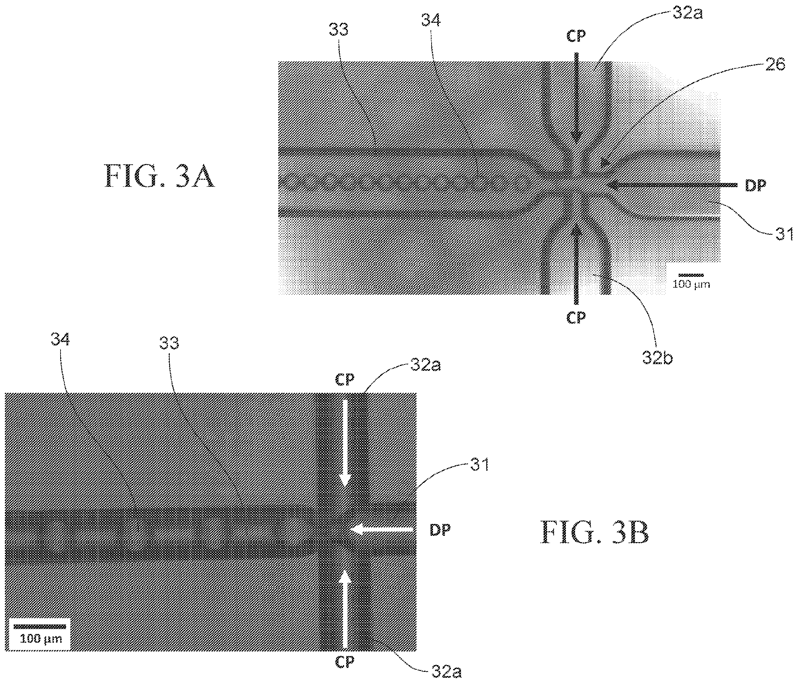

As will be discussed in more detail below, the disclosed systems and methods generate agglomeration-resistant monodispersed sirolimus microspheres of controlled size and internal morphology by microfluidic emulsification and subsequent solvent evaporation. The microfluidic emulsification uses step emulsification (Figures 1A-1E) and optionally flow-focusing junctions (Figures 2 and 3A-3C). The disclosed system and method generate stable droplets in dripping regime that can be maintained for 48 hours and result in droplets of highly consistent size created across all terraces of the microfluidic module and chip used in step microfluidic emulsification. In some embodiments, the droplet size in dripping regime can be controlled by the smallest dimension of droplet forming microchannels, which is typically the depth of microchannels. This confirms that droplet pinch-off can be driven by the gradient of capillary pressure experienced when a deformed droplet squeezed in a shallow microchannel enters a deep microfluidic well.

In some examples, the size of the generated microspheres can be accurately predicted from the dispersed phase formulation and the size of the parent droplets. In some embodiments agglomeration-resistant monodispersed sirolimus (SRL) microspheres with a diameter of 1.8-8.5 $\mu$m can be produced by microfluidic step emulsification and solvent evaporation using single crystal silicon chips consisting of 10 parallel arrays of terraced microgrooves. In some examples, a continuous generation of highly monodisperse (such as CV$\leq$3%, CV$\leq$4%, or CV$\leq$5%) droplets from micro-grooves with a depth of about 2-5 $\mu$m can be observed over 25 hours across all terraces. In some embodiments, the particle size after solvent evaporation can be reduced by about 10-15% during freeze-drying without any effect on the particle size uniformity. In some examples, the microspheres can be small enough and highly monodisperse to be safely injected in the blood stream using hypodermic needles of any size and can be used as drug delivery coatings for implantable medical devices, such as cardiac stents and balloon catheters.

The particle morphology can have an effect on drug release rate with less significant burst release expected to occur from microspheres with more homogeneous internal structure. As is presently disclosed, the drug-containing microspheres may be substantially amorphous.

Disclosed is a method for making a microsphere which contains only substantially amorphous drug. The microspheres are substantially free of polymer. Such particles may be useful, for example, as one method of achieving a faster dissolution of the particle and resulting in higher maximal concentrations of drug when the particles are delivered to a living body. The substantially polymer-free microsphere is advantageous as the foreign body response to the particle may be minimized in tissues that are sensitive to polymeric materials and their degradation byproducts.

The presently disclosed approach for the manufacturing of drug-loaded monodisperse microparticles can enable the development of new and more effective implantable drug delivery devices and improved methods for subcutaneous drug administration, which can lead to better therapeutic treatments. In particular, agglomeration-resistant monodispersed sirolimus microspheres without a polymer component which are generated by microfluidic emulsification / solvent evaporation can open up new routes for the manufacturing of more effective implantable devices and for the development of improved subcutaneous drug injections methods for restenosis treatment.

System for Making Microspheres

A system comprising a microfluidic module and chip may be used for producing microspheres that are monodispersed and have controllable particle sizes. In some embodiments, stable droplet production can be maintained for at least 24 hours, at least 36 hours, or at least 48 hours. Microfluidics can enable manipulation of fluids at the micrometer scale. Some advantages of microfluidics include the use of small reagent volumes, miniaturized devices, and low energy consumption. As well, microfluidics can produce high quantities of droplets and/or particles that are uniform/monodisperse in size.

**Figures 1A-1E** illustrate an embodiment of a microfluidic module and chip 10. **Figure 1A** illustrates a microfluidic rig 13a with two syringe pumps 11a and 11b - one for the continuous phase (11b) and one for the dispersed phase (11a). The pumps are fluidly connected to the module 13a such that the pump for the continuous phase 11b is fluidly connected with holes B (12b) and the pump for the dispersed phase 11a is fluidly connected with hole A (12a). In some embodiments, the microfluidic module can also include an emulsion collection syringe 11c. In some embodiments, a reflected-light microscope 14 can also be equipped in the microfluidic module for observation of droplet generation. In some embodiments, the microfluidic module is made of stainless steel.

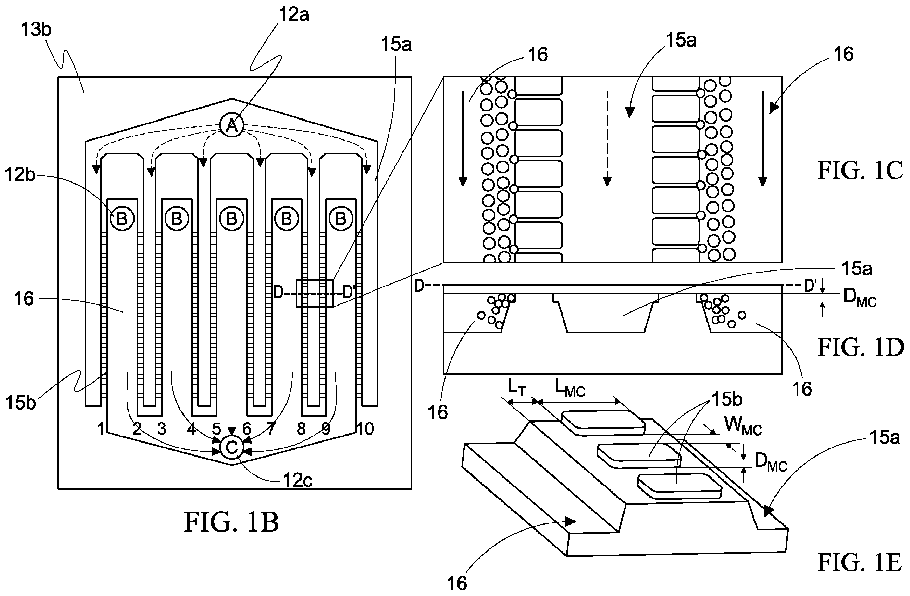

**Figure 1B** illustrates an embodiment of a microfluidic chip 13b for use with the microfluidic module 13a. As illustrated, in some embodiments, the microfluidic chip 13b can include 6 dispersed phase flow channels 15a and 10 parallel rows of terraced microchannels 15b, which are labeled with the terrace numbers 1-10. The chip of Figure **1B** can also include hole "A" which is an inlet hole for the dispersed phase 12a, holes "B" which provide inlet holes for the continuous phase 12b, and hole "C" which provides an outlet hole for the emulsion 12c. In some embodiments, the dispersed phase flow channels 15a can be dead-end channels filled with the dispersed phase composition and the continuous phase flow channels 16 are filled with the continuous phase composition. In some embodiments, the chip illustrated in Figure 1B can have a width of approximately 25 mm. In some examples, the chip illustrated in Figure 1B can have a height of between about 25 mm and about 28 mm. In some examples, the chip can have a distance of approximately 3.5 mm between the centerlines of adjacent inlet holes for the continuous phase (i.e., hole "B"). In some embodiments, the chip can have a distance of approximately 14.2 mm between the centerlines of the first and the fifth inlet holes for the continuous phase (i.e., hole "B"). In some examples, each of the cross-flow channels (i.e., continuous phase flow channels) can have a length of approximately 11.1 mm. In some embodiments, the microfluidic chip may be made of single crystal silicon.

Figure 1C illustrates a magnified top view of a single dead-end channel along cross-section "D-D'," **Figure 1D** illustrates a magnified side view of the dead-end channel, and **Figure 1E** illustrates a portion of the terrace wall with three microchannels 15b on the top and a deep cross-flow channel 15a and 16 at each side. The magnified side view of **Figures 1D-1E** illustrate the terraced microchannel (MC) depth ($D_{MC}$) while **Figure 1E** illustrates the MC width ($D_{MC}$), the MC length ($L_{MC}$), and the terrace length ($L_T$). The significance of each of these values will be discussed in more detail below. As will be discussed later, the size of the droplets/microspheres can be controlled by the dimensions of the microchannels that produce the droplets.

**Figure 2** illustrates a schematic of another embodiment of microfluidic module and chip 20. The microfluidic module can includes a first syringe pump 21 that contains an aqueous phase (i.e., continuous phase) and a second pump 22 that contains an oil phase (i.e., dispersed phase). The first and the second syringe pumps 21 and 22 provide the continuous phase composition and the dispersed phase composition, respectively, to the microfluidic chip 23. Microfluidic junctions 26 can be used on a microfluidic chip 23 to form oil-in-water emulsions. The oil droplets formed in the aqueous phase can then be dried to form microparticles. The microfluidic module can also include a microscope 24 (optionally with an integrated high-speed camera) to provide a real-time visualization of the formation of microdroplets. The microdroplets formed by the microfluidic module can then exit the microfluidic module where it can be collected 25.

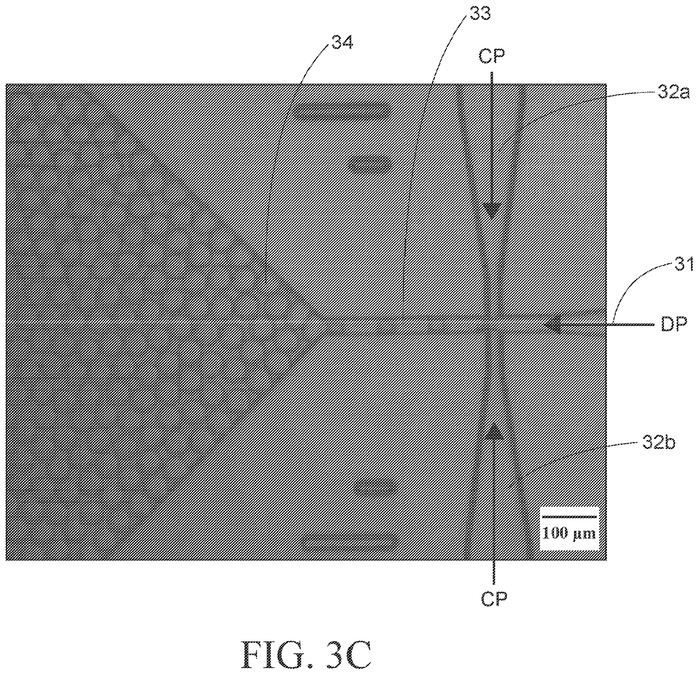

In some embodiments, the microfluidic chip can be configured to include one or more droplet forming junctions 26. In some embodiments, the junction microfluidic chips include 3D flow focusing junctions. As illustrated in **Figure 3A**, a droplet forming junction 26 includes a dispersed phase channel 31, two continuous phase channels 32a and 32b, and an outlet channel 33 that come together to form the droplet forming junction. The disperse phase channel 31 carries the dispersed phase (DP) solution toward the junction, the continuous phase channels 32a and 32b each carries the continuous phase solution toward the junction. When these solutions meet at the junction, the disperse phase was injected into the continuous phase stream to form a plurality of droplets 34 surrounded by the continuous phase. Figures 3B and 3C show additional droplet forming junction configurations. In some embodiments, the microfluidic chip may comprise one single droplet forming junction. In some embodiments, the microfluidic chip may be a multijunction chip. For examples, the microfluidic chip may comprise 2, 3, 4, 5, 6, 7, 8, 9, or 10 junctions.

This system can provide for the manufacturing of drug-loaded microparticles. For example, this system can be used to form drug-loaded microspheres/microparticles by injecting a dispersed phase containing the drug and an organic solvent into a continuous phase containing deionized (DI) water and a surfactant.

In some embodiments, the design of the microfluidic chip can affect the microparticle formation in addition to the emulsion formulation and the rate of solvent evaporation. For example, in the microfluidic chip illustrated in Figure 1B, the continuous phase to the dispersed phase flow ratio can exceed 1000. In some embodiments, the continuous phase to the dispersed phase flow ratio can exceed 60, 100, 200, 300, 400, 500, 600, 700, 800, 900, or 1000. This can ensure that the droplets are generated in a large pool of the continuous phase solution. For example, in embodiments where dichloromethane (DCM) is used as the solvent in the dispersed phase, the solubility of DCM in water is approximately 1.60% at 20 °C. As the continuous to dispersed phase flow ratio becomes greater than 62.5, DCM can be quickly removed from the droplets within the chip by extraction into the surrounding aqueous phase without any evaporation. In some examples, the quick removal of DCM from the droplets can result in more homogenous structure of drug-loaded particles than slow removal of DCM by evaporation from the particles stored in water. In other examples, such as the junction microfluidic chip illustrated in Figure 3A-3C, because of the 3D geometry of the channels, the disperse phase jet can be fully surrounded by the continuous phase during droplet formation. This can contribute to faster DCM removal as a result of the large contact area between DCM and water.

Method of Microparticle Production

Microspheres or microparticles containing a drug are produced using microfluidic step emulsification. A dispersed phase composition containing the drug and a solvent is injected into a continuous phase composition in a microfluidic chip to generate droplets containing the drug. The droplets are formed, suspended, and collected in the aqueous phase. In some embodiments, a collection syringe may be used to collect the droplets. In some embodiments, the solvent in the dispersed phase droplets is removed and extracted into the surrounding continuous phase (i.e., aqueous phase) solution. In some embodiments, the solvent further evaporates into the air from the continuous phase solution.

As the solvent in the droplets are removed, homogenous drug-loaded particles are formed in the continuous phase solution. In some embodiments, the particles can be formed less than about 24 hours to about 48 hours following the droplet productions. In some embodiments, the particles are further washed to remove the continuous phase solution before freeze drying. In some embodiments where a excipient is present in the droplets/particles, phase separation may occur about 48 hours after the droplet production without the wash cycle, which leads to the lack of uniformity of the particles.

In some embodiments, the dispersed phase (i.e., oil phase) comprises an organic phase solution. This organic phase solution can include a drug with an organic solvent. The drug can be, for example, sirolimus (SRL). In some embodiments, the organic solvent can be any solvent immiscible in water that is capable of dissolving the drug. For example, the solvent for SRL can include, but is not limited, dichloromethane (DCM), acetone, chloroform, methanol, ethanol, ethyl acetate, acetonitrile, or isopropyl acetate. In some examples, the continuous phase (i.e. aqueous phase) comprises a cross-flowing aqueous solution. The aqueous solution can include, for example, deionized (DI) water and a surfactant. In some embodiments, the surfactant in the continuous phase may be PVA, poly(vinyl pyrrolidone) (PVP), Tween 80, Triton X-100, sodium dodecyl sulfate (SDS), Pluronic surfactants, for example Pluronic F68 or Pluronic F127, or monomethoxypolyethylene oxide (MPEO)-b-PLA diblock copolymers. In some embodiments, cross-flowing aqueous solution can include, for example, poly (vinyl alcohol) (PVA).

In some embodiments, the microfluidic chips disclosed in Figures 1B and 3A-3C can be used to form microspheres of substantially pure sirolimus (i.e., substantially free of polymer). For example, the organic phase solution, which is a part of the dispersed phase composition, may comprise sirolimus and a solvent, which is injected through microchannels into the deep wells filled with cross-flowing aqueous solution of PVA as the continuous phase composition. In some embodiments, the solvent in the dispersed phase may be any solvent that is capable of dissolving sirolimus. For example, the solvent may be, but is not limited to, acetone, chloroform, methanol, ethanol, ethyl acetate, acetonitrile, or dichloromethane.

In some embodiments, microparticles can be formed using junction microfluidic chips that have 3D flow focusing junctions (e.g., Figure 3A-C). In some examples, the microfluidic chip can include a 3D design of flow-focusing orifice which can lead to faster solvent removal than in traditional flow-focusing chips with planar (2D) channels, because the continuous phase fully engulfs the dispersed phase jet during droplet generation.

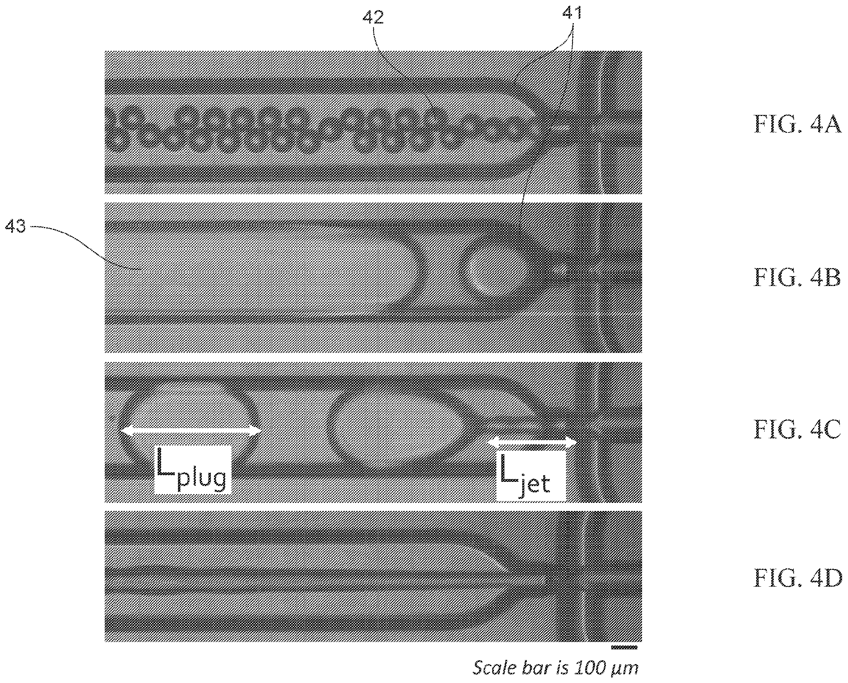

In some embodiments, droplets may be generated in junction microfluidic chips under a variety of regimes as illustrated in **Figures 4A-4D. Figure 4A** illustrates droplets 42 generation by squeezing, **Figure 4B** illustrates droplet generation by squeezing with plugs 43, **Figure 4C** illustrates droplet generation by dripping with plugs 43, and **Figure 4D** illustrates droplet generation by threading. In some embodiments, the plugs 43 are droplets with a diameter greater than the diameter of the outlet channel 41. In some embodiments, these droplets have a non-spherical shape and can occupy the whole cross-section of the outlet channel 41. In some embodiments, the microfluidic chip can form moving plugs in the outlet channel 41.

In some embodiments, the cross-flow microfluidic chips with microchannel arrays (as seen in Figures 1B-1D) may be used to scale up the production of droplets/particles. In some embodiments of the microfluidic chips, the organic phase solution (i.e., the dispersed phase) can comprise drug (such as sirolimus) and organic solvent (such as DCM), that is injected through microchannels into deep wells (e.g., Figure 1D) filled with cross-flowing aqueous solution of PVA (i.e., the continuous phase). In other embodiments, the continuous phase may be any of the aqueous solution of poly(vinyl pyrrolidone) (PVP), Tween 80, Triton X-100, sodium dodecyl sulfate (SDS), Pluronic surfactants, for example Pluronic F68 or Pluronic F127, or monomethoxypolyethylene oxide (MPEO)-b-PLA diblock copolymers.

In some embodiments, the continuous phase may contain at least 1 wt. % of the surfactant. In some embodiments, the continuous phase may contain about 1 wt % to about 2 wt %, about 1 wt % to about 1.5 wt % of the surfactant. For example, the continuous phase may contain at least 1 wt. % of PVA. In some embodiments, the continuous phase may contain about 1 wt % to about 2 wt %, about 1 wt % to about 1.5 wt % of PVA.

Because droplets are created in large reservoirs filled with the continuous phase solution, the solvent can be quickly extracted from the droplets into the surrounding aqueous phase leaving behind homogeneous spherical microparticles.

After the production of the droplets, the final product may be contaminated with PVA if the particles are not washed. PVA is generally recognized as safe (GRAS) by the FDA and the European Food Safety Authority (EFSA) has approved the use of PVA as a food additive if the total intake of PVA does not exceed 4.8 mg/kg bw/day. However, residual PVA may affect the bulk properties of the product. In some embodiments, the PVA concentration in the continuous phase may be minimized in order to reduce the initial level of PVA contamination and the number of washing cycles required. In some embodiments, the microparticles may be washed at least 5 times. In some embodiments, the microparticles may be washed 6-10 times. In some embodiments, the microparticles may further be freeze dried.

In some embodiments, the generation of microspheres is caused by the established difference in Laplace pressure ($p_1 - p_2$). The difference in Laplace pressure can induce a sudden flow of the dispersed phase into the well and the dispersed phase jet breaks via a Rayleigh-Plateau instability. During droplet generation, the dispersed phase can expand on a terrace of the microfluidic chips into large discs. In some embodiments, if the contact angle between the dispersed phase and the channel wall on the terrace is low, the Laplace pressure on the terrace can be calculated as follows:

$$p_1 = \gamma \left( {}^1\!/_{r_1} + {}^2\!/_{D_{MC}} \right)$$

In the formula for Laplace pressure provided above, $\gamma$ is the interfacial tension and $r_1$ is the droplet radius on the terrace in the downstream direction. In some examples, the confined droplets can expand on the terrace due to an influx of the dispersed phase fluid. However, in some embodiments, the $p_1$ does not change because:

$$D_{MC} \ll r_1; \text{ and } p_1 \approx {}^{2\gamma}\!/_{D_{MC}}$$

In some embodiments, as $p_1$ does not change, the Laplace pressure on the terrace does not depend on droplet size but only on the channel (terrace) depth. Once the dispersed phase reaches the terrace edge and enters a deep well, the droplet generated can expand in the well in all-directions as it is no longer confined in the orthogonal direction. In some embodiments, the Laplace pressure in the well can be:

$$p_2 = {}^{\gamma}\!/_{r_2}$$

In the formula for Laplace pressure provided above, $r_2$ is the radius of the interface in the well, which is much greater than $D_{MC}$ and thus, $p_1 \gg p_2$.

The droplet can therefore be generated through Laplace pressure induced snap-off. A confined droplet with a height $D_{MC}$ can expand on the terrace but once it reaches the terrace edge, the interface can be quickly pulled into the well, where it releases a droplet and then retraces back. This pinch-off mechanism requires no viscous interaction and can occur due to changes in the curvature of the interface, affecting the Laplace pressure. This can be demonstrated by varying the continuous phase flow rate in dripping regime. In some embodiments, this varying of the continuous phase may have no noticeable impact on the droplet size. In some examples, the droplet size was not impacted even when the continuous phase flow rate was very low or stopped entirely. In some embodiments, when the continuous phase flow rate is low or stopped, the generated droplets can accumulate on the cover glass and self-assemble into densely packed regular hexagonal arrays.

In some embodiments, a clean hydrophilic surface of the silicon microfluidic chip may be advantageous after usage (in some embodiments, the chip is reusable). In some embodiments, a clean silicon surface is hydrophilic because the presence of polar silanol (-Si-OH) groups. In some examples, after emulsion production, the silicon wall can be covered with organic contaminants deposited during the production process. This can include, for examples, sirolimus, and PVA which can lead to more hydrophobic surface that can compromise droplet generation. In some embodiments, organic contaminants can be partially removed by ultrasound treatment of the chip by a household detergent solution. In some embodiments, a chemical treatment of the chip may be performed after the ultrasound treatment, which can involve oxygen or ozone plasma oxidation of organic contaminants in a plasma reactor.

Hydrophilic chip surfaces can keep a low contact angle between organic droplets and channel walls in the chip. Since the step emulsification process is based on Laplace pressure difference and curvature imbalance along the droplet interface, properly restoration of hydrophilic surfaces can allow uniform droplets to be generated in the chip continuously for at least 24 hours.

In some embodiments, the microfluidic chips can be cleaned by DCM and/or furnace treated to remove residue prior to being used or reused. In some embodiments, about 90% of residue can be removed after furnace treatment. In some embodiments, the microfluidic chips can be cleaned by ultrasound treatment of the microfluidic chip by a household detergent solution. In some embodiments, a chemical treatment of the chip may be performed after the ultrasound treatment, which can involve oxygen or ozone plasma oxidation of organic contaminants in a plasma reactor.

In some embodiments, surface treatment using the previously discussed procedure can lead to sustained droplet generation for at least 24 hours for all runs. In some examples, any wetting of the terrace walls by the dispersed phase can reduce Laplace pressure on the terrace ($p_1$), as a result of flattening the dispersed phase interface in the orthogonal direction, as the radius of curvature can become greater than $D_{MC}$ and approaches infinity for the wetting angle 90°. In some embodiments, this can lead to reduced driving force for droplet pinch-off and may cause a continuous outflow of the dispersed phase from the microchannels and formation of large polydisperse droplets. In some embodiments, although the terrace wall wettability was slightly altered after 24 hours compared to the case after one hour due to prolonged contact of silicon surface with emulsion ingredients (DCM, SRL and PVA), no change in droplet size was observed.

*Droplet Sizes*

In **Figures 4C,** the length of the plug ($L_{plug}$) is the distance between the upstream and downstream edge of a plug. In some examples, the length of the jet ($L_{jet}$) can be the length of a thread connecting newly formed droplets and the dispersed phase stream emerging from the orifice. In some embodiments, the $L_{plug}$ and the $L_{jet}$ can be controlled by the flow rate of the dispersed phase. In some embodiments, the higher the flow rate of the dispersed phase, the higher the $L_{plug}$ and the $L_{jet}$. In some examples, the plug and jet formations are discouraged as they can lead to polydisperse droplets and particles.

Droplet generation in a junction microfluidic chip can be estimated by calculating the Weber number of the dispersed phase ($We_{DP}$):

$$We_{DP} = \frac{\rho_{DP} * u^2_{DP} * W}{\sigma}$$

As well, the capillary number of the continuous phase can also be calculated ($Ca_{cp}$):

$$Ca_{cp} = \frac{u_{CP} * \mu_{CP}}{\sigma}$$

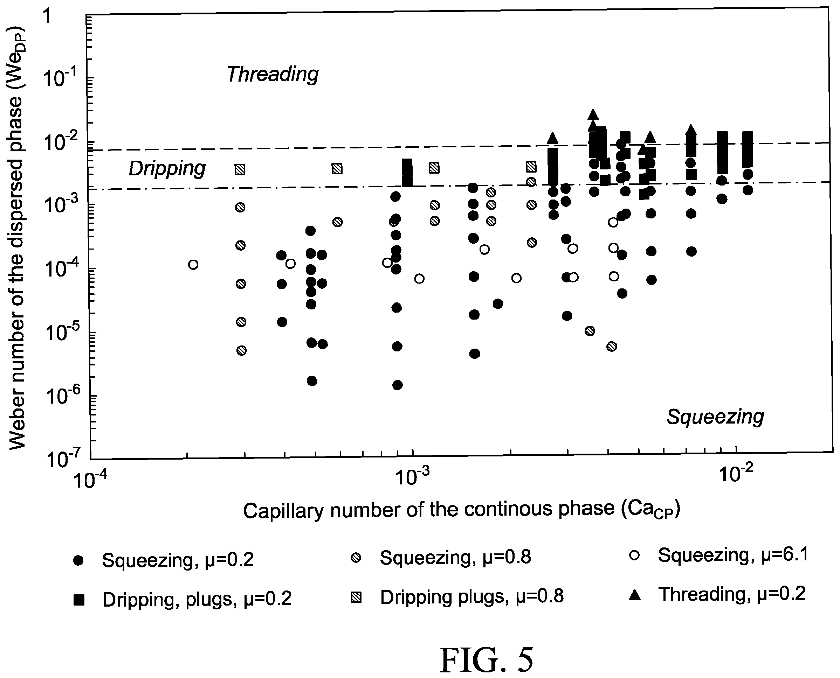

In the formulae provided above, $\rho$ = *density,* $u$ = velocity; $\mu$ = viscosity; $W$ = channel width; and $\sigma$ = interfacial tension. In some embodiments, the values of the $We_{DP}$ and the $Ca_{cp}$ can affect the size of the droplets. In some examples, uniformly sized droplets can be generated only if both the $We_{DP}$ and the $Ca_{cp}$ are below their critical values. An example of critical values for the $We_{DP}$ are shown by the lower dashed line in Figure 5.

In some embodiments, the droplet size can be adjusted by controlling the capillary number of the continuous phase ($Ca_{cp}$). In some examples, the higher the value of the $Ca_{cp}$, the smaller the resulting droplet diameter (providing all other parameters are the same). In some embodiments, if the $Ca_{cp}$ is above the critical value, a jetting of the dispersed phase can occur and may result in the formation of polydisperse droplets.

**Figure 5** illustrates a graph of the Weber number of the dispersed phase ($We_{DP}$) to the continuous phase ($Ca_{cp}$) for threading, dripping, and squeezing. The viscosity ($\mu$) for each of the methods of generating droplets was subsequently calculated by dividing the viscosity of the dispersed phase ($\mu_{DP}$) by the viscosity of the continuous phase ($\mu_{CP}$):

$$\mu = \frac{\mu_{DP}}{\mu_{CP}}$$

As illustrated, the $\mu_{squeezing}$ ranges between 0.2 and 6.1; the $\mu_{dripping,plugs}$ ranges between 0.2 and 0.8, and the $\mu_{threading}$ was calculated to be about 0.2.

In some embodiments, the CV can be calculated as:

$$CV = \frac{\sigma}{\overline{D}_d} x 100$$

where $\sigma$ is the standard deviation of 30 droplet diameters and $\overline{D}_d$ is the average droplet diameter. The micrographs of the collected droplets and particles can be analysed using ImageJ software. The particle diameter can be predicted from $\overline{D}_d$ based on the mass balance of non-volatile solids in the dispersed phase:

$$D_{p,p} = \overline{D}_d \sqrt[3]{x_s \frac{\rho_p}{\rho_d}}$$

where $D_{p,p}$ is the predicted particle diameter, $x_s$ is the mass fraction of non-volatile solids in the dispersed phase, and $\rho_p$ and $\rho_d$ are the density of particles and droplets, respectively.

In cross-flow microfluidic chips with terraced microchannels, the microfluidic chips may comprise two sets of cross-flow channels that delivers dispersed phase solution and continuous phase solution. Parallel rows of terraced microchannels connects the two sets of cross-flow channels where the dispersed phase solution is injected into the continuous phase solution to from a plurality of droplets. The droplets may be produced by a dripping regime in these cross flow microchips with terraced microchannels (MC).

In these embodiments, the mean droplet size in the dripping regime can be controlled by the MC depth, $D_{MC}$. As illustrated in **Figure 1D,** the $D_{MC}$ is the thickness of the microchannel formed on the terrace of the microfluidic chip. In some embodiments, the mean droplet sized may be between about 5 $\mu$m and 25 $\mu$m when the $D_{MC}$ was from about 2 $\mu$m to about 5 $\mu$m. In some embodiments, the ratio of the mean droplet diameter ($\overline{D}_d$) to the hydraulic channel diameter ($d_h$) for the channel depths of about 4 $\mu$m to about 5 $\mu$m ranged between about 3.3 to about 3.4. This ratio is similar to the droplet size to pore size ratio in Shirasuporous-glass (SPG) membrane emulsification. In some embodiments, the SPG membrane emulsification, the droplet size in the dripping regime can be proportional to the pore size of SPG membrane. This can mean that the droplet size to pore size ratio has a constant value which depends on the emulsion formation. In some embodiments, the droplet size to pore size ratio in SPG emulsification can be between 3 and 4. This can provide control of droplet size by selecting SPG membrane with a corresponding pore size. In some embodiments, because the pore-size of SPG membrane can range from between about 0.1 $\mu$m to about 40 $\mu$m, this can allow the preparation of emulsions with a mean droplet size ranging from between about 0.3 $\mu$m and about 160 $\mu$m. In some embodiments, the mean droplet size may be between about 0.4 $\mu$m and about 150 $\mu$m, between about 0.4 $\mu$m and about 120 $\mu$m, or between about 1 $\mu$m and about 100 $\mu$m.

In some embodiments, the cross-flow microfluidic chips can include grooved microchannels with trapezoidal cross-sections without any terraces ($L_T = 0$). In some embodiments, in microfluidic chips without a terrace, the ratio of the mean droplet diameter to the hydraulic channel diameter ($\overline{D}_d/d_h$) can be 3.0. In some examples, where the microfluidic chip includes a terrace (i.e., $L_T > 0$), the $\overline{D}_d/d_h$ can range above 3 to nearly 6.

*Washing of Microspheres*

In some embodiments, after the microspheres are produced by step microfluidic emulsification, the solvent is removed from the microsphere into the surrounding aqueous phase. In some embodiments, the microspheres can be washed between 24-48 hours after production.

After the production of the droplets, the formed microspheres can be contaminated with the surfactant in the continuous phase, such as PVA, if the particles are not washed. Although PVA is generally recognized as safe, residual PVA can affect the bulk properties of the microspheres. In some embodiments, the PVA concentration in the continuous phase can be minimized in order to reduce the initial level of PVA contamination and the number of washing cycles required.

In some embodiments, washing the microspheres to remove PVA can affect the particle interactions with cell membranes in the body by increasing the hydrophilicity of the surface. In some examples, the microspheres can be washed by centrifuging the emulsion. The supernatant of the centrifuged emulsion can then be removed and replaced with an equal volume of aqueous solution. In some embodiments, the emulsion can then be vortexed.

The amount of residual PVA adsorbed on the particle surface can depend on the miscibility of the solvent blend with water. A higher amount of adsorbed PVA can be expected in the particles prepared from the solvent blend with a higher amount of isopropyl acetate (IPAc). In some examples, this can be due to higher aqueous solubility of IPAc compared to DCM. In some embodiments, when droplets are formed in a PVA solution, the PVA can be absorbed to the droplet surfaces in such a way that hydrophobic acetate tails of PVA molecules can be immersed in the organic phase, while hydrophilic alcohol groups will be exposed to the aqueous phase. In some embodiments, after solvent evaporation, the PVA coating can remain on particle surfaces and enhance hydrophilic properties of the particles because of exposed - OH groups on the surface. In some embodiments the increased absorption of residual PVA can result in increased hydrophilicity of the particles.

EXAMPLES

Examples 1-4 are not according to the claimed invention.

Example 1: Preparation of SRL-PLGA Microspheres

Disclosed is the production of agglomeration-resistant monodispersed sirolimus-loaded PLGA microparticles by step microfluidic emulsification. The microparticles are fabricated with tuneable size and internal morphology using single-crystal silicon chips with grooved microchannel arrays. The fabricated particles can be used for subcutaneous sirolimus administration or as drug micro-reservoirs in sirolimus-coated balloons, thereby offering new opportunities for the development of improved restenosis treatments or other pharmacologic therapies based either on subcutaneous drug injections or on implantable drug delivery systems.

As discussed, microspheres can be generated using microfluidic chips with terraced microchannels wherein the microfluidic chips comprise two sets of cross-flow channels that deliver dispersed phase solution and continuous phase solution. Three cross flow silicon microchips, described in Table 1 below, with terraced microchannels (MCs) arranged in 10 parallel arrays may be used for droplet generation. **Figure 1B** illustrates an example of the terraced microchannel microchips in 10 parallel arrays. Table 1 provides an example of the dimensions of various microfluidic chips as shown in **Figure 1E.**

Table 1.

| CHIP | CMSC-1 | CMS6-2 | CMS6-3 |
|---|---|---|---|
| $D_{MC}$ ($\mu m$) | 5 | 4 | 2 |
| $W_{MC}$ ($\mu m$) | 18 | 8 | 12 |
| $d_h$ ($\mu m$) | 7.0 | 4.1 | 3.3 |
| $L_{MC}$ ($\mu m$) | 140 | 140 | 140 |
| $L_T$ ($\mu m$) | 60 | 30 | 30 |
| $N_{MC}$ | 540 | 1850 | 1710 |
| $n_{MC}$ | 54 | 185 | 171 |

$D_{MC}$ ($\mu m$) = **MC** depth; $W_{MC}$ ($\mu m$) = MC width; $d_h$ ($\mu m$) = hydraulic diameter of MCs; $L_{MC}$ ($\mu m$) = **MC** length; $L_T$ ($\mu m$) = terrace length; $N_{MC}$ = total number of MCs: $n_{MC}$ = number of MCs in a single row.

The used chips can be washed in an ultrasound bath set at 10% power using a mild detergent dissolved in a mixture of water and ethanol. The chip can then be rinsed with Milli-Q water, oven dried, and transferred to a plasma cleaner to oxidize persistent organic contaminants that could not be removed by the detergent. In some examples, the plasma oxidation can be performed at high vacuum of $1\times10^{-5}$ mbar for about 15 min (pure $O_2$) or about 20 min (about 25% O2 and about 75% Ar). The clean microfluidic chip can then be placed on a watch glass in a beaker containing the continuous phase and sonicated for about 20 min. The continuous phase can then be replaced, and the chip stored in the fresh continuous phase. To evaluate the cleaning process, the contact angle between a water droplet and a dry chip surface was measured. In some embodiments, the contact angle of a water drop with a contaminated silicon surface can be approximately 60°. After washing with dichloromethane (DCM), the contact angle was found to be reduced to 32.4° as a result of the partial removal of organic contaminants. After the microfluidic chip was treated by plasma oxidation, the contact angle was further reduced to 16.9° as a result of the complete removal of persistent organic layer.

The surface treatment using the previously discussed procedure can lead to sustained droplet generation for at least 24 hours for all runs. A long-term droplet production stability in the CMS6-2 chip was compared in runs S2 and S5 in **Table 2** provided below.

| Table 2. The experimental conditions, emulsion formulations, and droplet/particle sizes for different sample runs. | | S1 | S2 | S3 | S4 | S5 | S6 | S7 | S8 | S9 |
|---|---|---|---|---|---|---|---|---|---|---|
| Nominal channel depth. $D_{MC}$ ($\mu m$) | | 5 | 4 | 2 | 4 | 4 | 4 | 4 | 4 | 4 |
| Dispersed phase composition (wt%) | SRL | 1.32 | 1.32 | 1.32 | 1.32 | 1.64 | 1.76 | 1.80 | 1.84 | 2.25 |
| | PLGA | 2.68 | 2.68 | 2.68 | 2.68 | 2.34 | 2.24 | 2.20 | 2.16 | 2.25 |
| | IPAc | 72.00 | 72.00 | 72.00 | 72.00 | 72.00 | 72.00 | 72.00 | 72.00 | 71.63 |
| | DCM | 24.00 | 24.00 | 24.00 | 24.00 | 24.00 | 24.00 | 24.00 | 24.00 | 23.88 |
| Continuous phase composition (wt%) | PVA | 1.50 | 1.50 | 1.50 | 1.50 | 1.50 | 1.00 | 1.00 | 1.00 | 1.00 |
| | Milli-Q water | 98.50 | 98.50 | 98.50 | 98.50 | 98.50 | 99.00 | 99.00 | 99.00 | 99.00 |
| Drug loading (wt%) | | 33 | 33 | 33 | 33 | 42 | 44 | 45 | 46 | 50 |
| Total solids in the dispersed phase (wt%) | | 4 | 4 | 4 | 4 | 4 | 4 | 4 | 4 | 5 |
| Droplet production time | | 48 h | 24 h | 24 h | 24 h | 48 h | 24 h | 24 h | 24 h | 24 h |
| Flowrate (mL/h) | Dispersed phase | 0.15 | 0.03 | 0.01 | 0.03 | 0.05 | 0.02 | 0.05 | 0.02 | |
| | Continous phase | 1.00 | 1.00 | 1.00 | 1.00 | 1.00 | 1.00 | 1.00 | 1.00 | 1.00 |

(continued)

| Table 2. The experimental conditions, emulsion formulations, and droplet/particle sizes for different sample runs. | | | | | | | | | |
|---|---|---|---|---|---|---|---|---|---|
| | S1 | S2 | S3 | S4 | S5 | S6 | S7 | S8 | S9 |
| Mean droplet size ($\mu$m) | 23.5 | 13.8 | 5.2 | 13.8 | 13.8 | 13.8 | 13.6 | 13.8 | 13.5 |
| CV of droplet sizes (%) | 3.0 | 2.5 | 3.5 | 2.5 | 3.0 | 2.6 | 2.0 | 2.5 | 2.4 |
| Predicted particle size ($\mu$m) | 7.0 | 4.1 | 1.5 | 4.1 | 4.2 | 4.2 | 4.3 | 4.3 | 4.5 |
| Mean particle size ($\mu$m) | 8.5 | 3.8 | 1.8 | 4.2 | 4.0 | 4.2 | 4.2 | 4.2 | 4.3 |
| CV of particle sizes (%) | 4.5 | 5.2 | 6.0 | 5.2 | 4.1 | 4.3 | 5.1 | 4.8 | 4.6 |

In some embodiments, the mean droplet size after 24 hours and 48 hours was the same (13.8 $\mu$m), but the CV increased slightly from about 2.5% after 24 hours to about 3% after 48 hours.

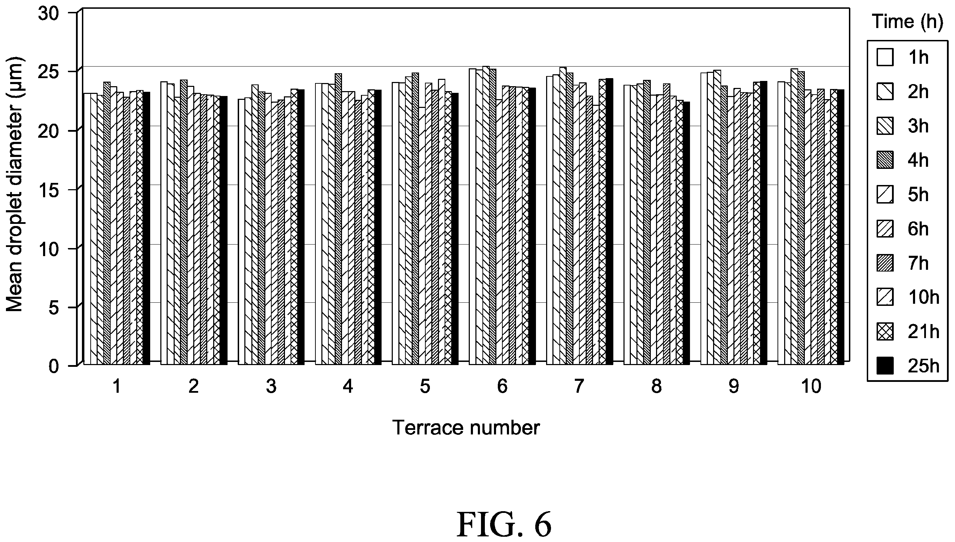

**Figure 6** illustrates the temporal fluctuations of droplet sizes generated from the different terraces of the CMS6-1 chip described in **Table 1**. As shown in **Figure 6,** the droplet size fluctuations between different terraces can be below 1.5 $\mu$m and the mean droplet size of 23.5 $\mu$m can be stable over time. As shown, about 89-95% of all channels can be actively producing droplets at any time. As well, the droplet generation regime can shift from a dripping regime (i.e., small and uniform drops) to a continuous out-flow regime (i.e., large and non-uniform drops) at the critical dispersed phase velocity. In some embodiments, droplet generation can depend on channel geometry, interfacial tension, and viscosity ratio between the two phases. In some examples, when triolein droplets were generated in an aqueous solution of about 1 wt% SDS, the critical velocity in grooved channels with similar geometry was 2.2 mm/s. In the S1 run described in **Table 2** below, the average dispersed phase velocity in the microchannels was 4.2 mm/s. In some embodiments, the critical velocity can be higher than 2.2 mm/s, because DCM is less viscous than triolein. In some examples, the critical velocity can be higher at lower dispersed phase to continuous phase viscosity.

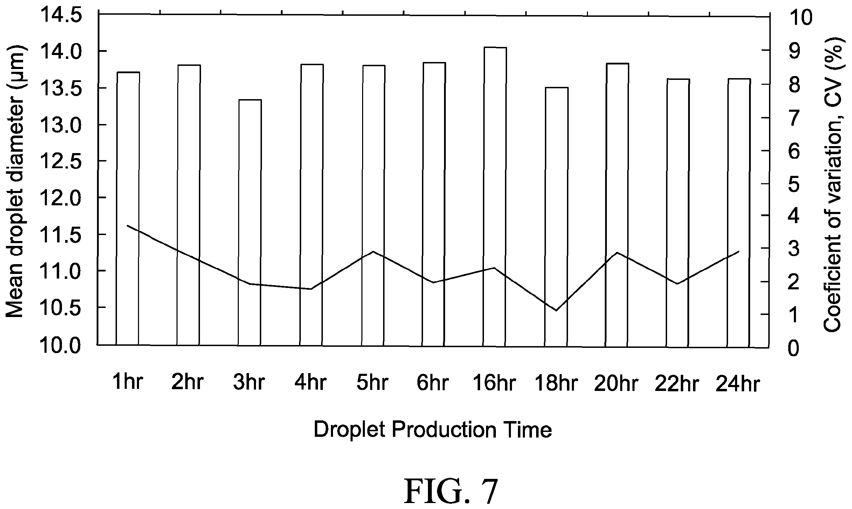

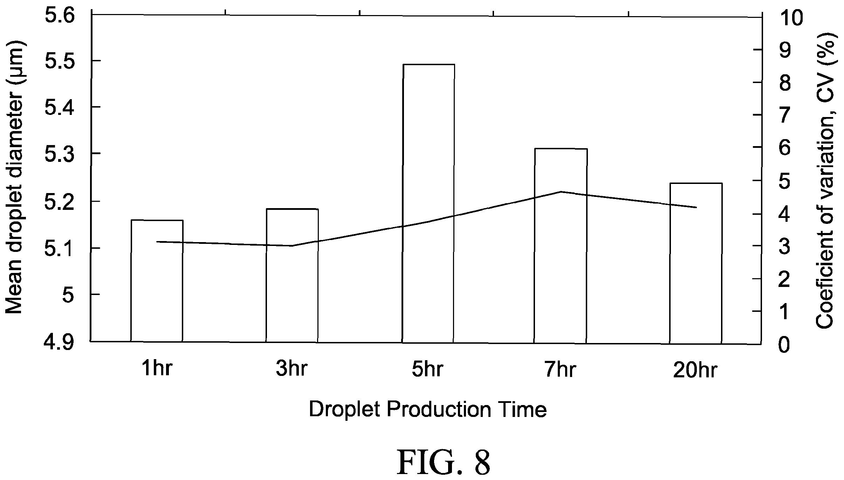

**Figure 7** and **Figure 8** also illustrate temporal droplet size stability on the CMS6-2 chip and the CMS6-3 chip respectively. In some embodiments, because of the smaller channel dimensions in the CMS6-2 chip (**Figure 7**) and the CMS6-3 chip (**Figure 8**), droplet sizes were lower than in the CMS6-1 chip (**Figure 7**). For the CMS6-2 chip (**Figure 7**), the 90% confidence interval of the mean droplet size was (13.8 $\pm$ 0.6) $\mu$m with CV = 2.5%. According to the National Institute of Standards and Technology, particles may be considered agglomeration-resistant monodispersed if at least 90% of them have a size within 5% of the median size, $\overline{D}_d$. In some examples, for a normal size distribution, about 90% of the particle sizes are within 1.64 standard deviations ($\sigma$) of the mean. Droplets can therefore be monodispersed if $1.64\sigma \leq 0.05\overline{D}_d$, or CV $\leq$ 3%. Therefore, the 90% confidence limits of the mean for monodisperse droplets can be (13.8 $\pm$ 0.7) $\mu$m. In some embodiments, in the CMS6-3 chip (**Figure 8**), the 90% confidence interval of the mean droplet size can be (5.2 $\pm$ 0.3) $\mu$m with CV = 3.5%. In some examples, a CV value slightly above 3% can be attributed to larger error in estimating the size of smaller droplets.

After the microspheres are produced by step microfluidic emulsification, the solvent is removed from the microsphere into the surrounding aqueous phase. As shown in examples S5 and S6 in **Table 2,** in some examples, the PVA concentration in the aqueous phase can be reduced from about 1.5 wt% to about 1 wt% without noticeable impact on the mean droplet size and CV value. In some examples, the reduction in PVA concentration below 1 wt% can have detrimental effects on the droplet size uniformity.

The microspheres can be washed to remove the PVA as PVA can affect the bulk properties of the product. The emulsion can be collected in an open syringe and the particles were formed upon DCM evaporation from the droplets. The residual PVA may then be removed from the suspension through multiple washing cycles. Each washing cycle can consist of centrifuging the suspension at 3500 rpm for 10 min, removing the supernatant with a Pasteur pipette, adding an equal volume of about 0.05 wt% Tween 20 aqueous solution, and vortexing for 5-10 s. The concentration of residual PVA in the supernatant can be measured using a spectrometer. After a final wash, the level of supernatant in the vial can be reduced just above the level of sediment and the particles were dried using a benchtop freeze dryer. The vial content can be first frozen to about -20 °C and then left to dry at a pressure in the chamber of about 16 Pa and a condenser temperature of about -86.3 °C.

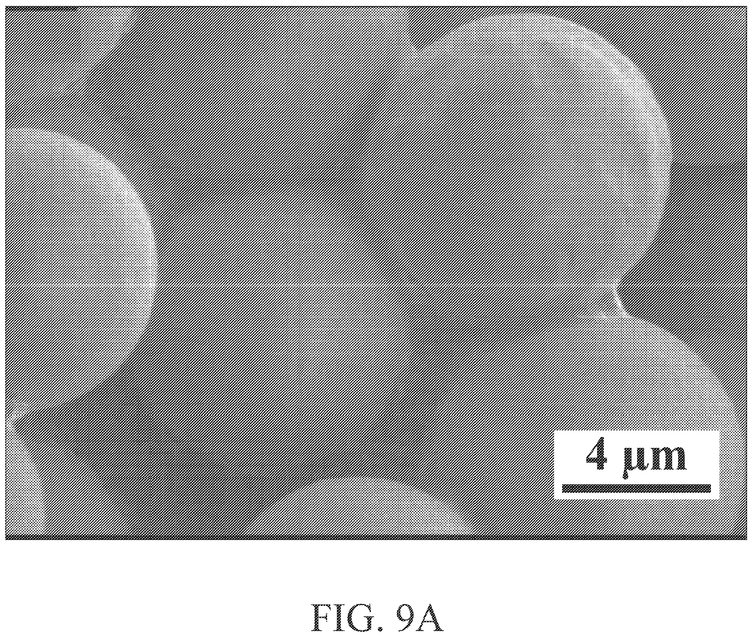

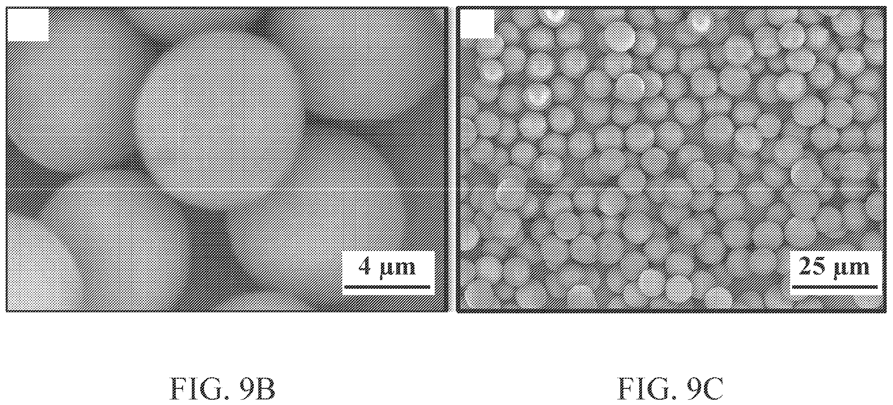

**Figure 9A** is a SEM image of SRL-loaded PLGA particles without washing. The unwashed particles can aggregate due to binding by the PVA film deposited between dried particles. As well, the formation of particle clusters by PVA bridges can alter all particle size-dependent properties of the product, such as syringeability, dispersibility in secondary polymer matrix, and drug release behavior. **Figures 9B-9C** are SEM images of the same sample after 8 washes. After 8 washes, the particle surface can be clean and smooth, without any visible PVA film. In some embodiments, even after many washes, a small fraction of PVA can still remain adsorbed to the particle surface because PVA forms an interconnected network with PLGA. However, the amount of molecularly bound PVA remaining is too small to be quantified and does not affect the particle size distribution.

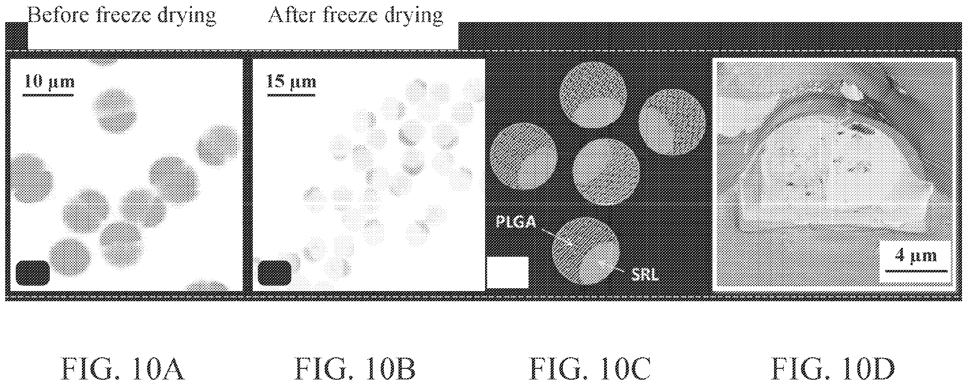

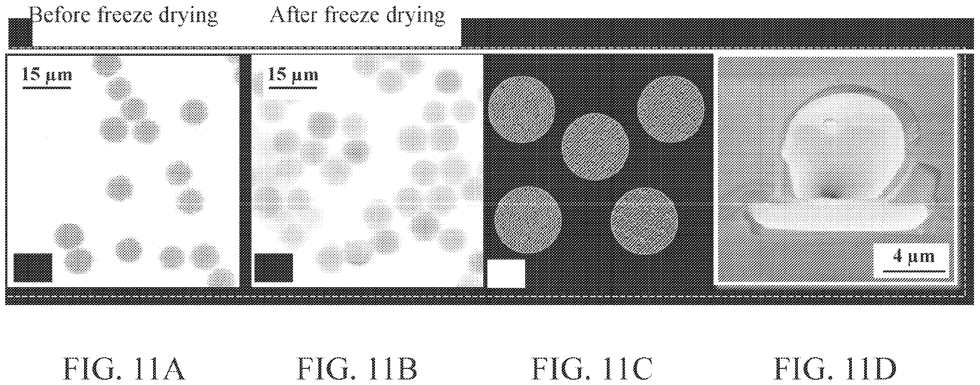

In addition to washing the microspheres to prevent phase separation, the rate in which solvent is removed can impact the amount of drug-polymer separation and whether there is uniform drug distribution within the polymer matrix. The solvent removal rate from the microspheres can be controlled by pre-saturation of the continuous phase with IPAc. **Figures 10A-10D** and **11A-11D** illustrate the effect of saturating the continuous phase with organic solvent (IPAc) on particle morphology in Sample S1 (from **Table 2**). Two different batches of sample S1 - S1A and S1B - were produced using PVA solutions with different levels of solvent saturation. **Figures 10A-10D** illustrate microspheres from sample S1A and **Figures 11A-11D** illustrate microspheres from sample S1B. For batch S1A, the continuous phase was about 1.5 wt% PVA solution pre-saturated with IPAc. In batch S1B, the continuous phase was pure aqueous solution with the same PVA concentration, thereby enabling higher initial rates of organic solvent extraction from droplets. **Table 3,** provided below, provides the mean particle size before and after freeze drying. The continuous phase was about 1.5 wt% PVA saturated with IPAc (S1A) or pure about 1.5 wt% PVA solution (S1B):

| Table 3. The mean particle size before and after freeze drying | | | | |
|---|---|---|---|---|
| | Before Freeze Drying | | After Freeze Drying | |
| Sample | Mean ($\mu m$) | CV (%) | Mean ($\mu m$) | CV (%) |
| S1A | 7.5 | 5.3 | 6.7 | 4.3 |
| S1B | 9.6 | 6.2 | 8.1 | 5.0 |

In both S1A and S1B, the particle size was above about 7 $\mu m$, but larger particles were formed by faster solvent removal rate in the S1B batch. **Figures 10A-10B** and **11A-11B** show CLSM images of particles before and after freeze drying. **Figures 10C** and **11C** show schematic diagrams of particle structures. **Figure 10D** and **11D** show SEM images of particles cross-sectioned by focused ion beam (FIB). The brighter particle domains are rich in sirolimus (SRL) and the darker domains are rich in PLGA. Nile red shows a preferential absorption toward SRL over PLGA due to the highly hydrophobic nature of SRL with an octanol/water partition coefficient (log P) of 4.3. Nile Red is a hydrophobic compound whose fluorescence is strongly influenced by the polarity of its environment. As a result, SRL-rich regions are less polar than PLGA-rich regions.

As shown in **Figures 10A-10D** and **11A-11D,** sirolimus is observed to be more uniformly distributed in **Figures 11A-11D** (Sample S1B) than in **Figures 10A-10D** (Sample S1A) as a result of pre-saturating the continuous phase with IPAc. **Figures 10B-10C** illustrates the formation of Janus particles with a single crescent-moon-shaped SRL-rich region on one side of the particle. As the sirolimus regions occupy smaller particle volume than PLGA-rich regions, the observed drug-polymer separation shown in **Figure 10B-10C** is consistent with a drug loading of about 33% sirolimus in Sample S1. (See column S1 in **Table 2). Figure 10D** illustrates that, because the phase separation is incomplete, both regions contain both components. As shown in the FIB-SEM image of **Figure 10D,** drug micro-patches are embedded within a PLGA domain, while SRL domain contains PLGA patches. In contrast, pre-saturating the continuous phase with IPAc can provide for faster solvent removal from droplets that can reduce drug-polymer separation and therefore lead to the incomplete segregation of SRL and PLGA. Unlike **Figures 10A-10D,** no boundary between the drug and the polymer was observed in **Figures 11A-11D.** As shown in **Figures 11A-11D,** the faster solvent removal can lead to more uniform distribution of drug in the solid matrix.

Example 2: Characterization of SRL-PLGA Microspheres

**Table 2,** provided above, summarizes emulsion formulations, fluid flow rates, the droplet-particle sizes, and their coefficient of variance (CV) for different sample runs.

As shown in **Table 2** provided above, a long-term droplet production stability in the CMS6-2 chip was compared in runs S2 and S5 in **Table 2.** As discussed above, in some embodiments, the mean droplet size after 24 hours and 48 hours was the same (13.8 $\mu m$), but the CV increased slightly from about 2.5% after 24 hours to about 3% after 48 hours.

In some embodiments, the mean particle size generated from the systems disclosed herein may be between about 10 $\mu m$ and about 1 $\mu m$ for the channel depths of between 6 $\mu m$ and about 1 $\mu m$. In some examples, the mean particle size can range between about 1 $\mu m$ and about 2.0 $\mu m$, between about 2 $\mu m$ and about 3.0 $\mu m$, between about 3.0 $\mu m$ and about 4.0 $\mu m$, between about 4.0 $\mu m$ and about 5.0 $\mu m$, between about 5.0 $\mu m$ and about 6.0 $\mu m$, between about 6.0 $\mu m$ and about 7.0 $\mu m$, between about 7.0 $\mu m$ and about 8.0 $\mu m$, between about 8.0 $\mu m$ and about 9.0 $\mu m$, between about 9.0 $\mu m$ and about 10.0 $\mu m$, and any value in between those ranges listed, including endpoints. In some embodiments, the mean particle size can have a CV of particle size ranging between 1% and 5%. In some examples, the CV of particle size can range between about 1.0% and about 1.5%, between about 1.5% and about 2.0%, between about 2.0% and about 2.5%, between about 2.5% and about 3.0%, between about 3.0% and about 3.5%, between about 3.5% and about 4.0%, between about 4.0% and about 4.5%, between about 4.5% and about 5.0%, and any value in between those ranges listed, including endpoints. In some embodiments, the SRL-PLGA particles can be loaded with drug with a weight % of between about 30% and about 50%. In some embodiments, the SRL-PLGA can have a drug weight% with a range between about 30% and about 35%, between about 35% and about 40%, between about 40% and about 45%, between 45% and about 50%, and any value in between those ranges listed, including endpoints.

In the study conducted and illustrated in **Table 2,** the mean particle size in sample S1 was about 8.5 $\mu$m, and about 95% of the particles range between 7.8-9.2 $\mu$m. These particles can be injected through a 31-gauge needle (or thinner). In some embodiments, the samples S2 and S3 can be suitable for the intravenous delivery of sirolimus. As provided in **Table 2,** even the biggest particles in the formulation presently disclosed are still several times smaller than the lumen of the smallest arteries (e.g., approximately 30 $\mu$m).

The mean droplet sizes in various chips were measured in regular time intervals over 20-25 hours with the results shown in **Figures 6-8. Figure 6** illustrates the mean droplet diameter in the CMS6-1 chip at different terraces over 25 hours. **Figure 7** illustrates the mean droplet diameter (in bars) and the coefficient of variation, CV (in solid line) in the CMS6-2 chip over 24 hours. **Figure 8** illustrates the mean droplet diameter (in bars) and the CV (in solid line) in the CMS6-3 chip over 20 hours. As shown in **Figure 6,** the droplet size fluctuations between different terraces can be below 1.5 $\mu$m and the mean droplet size of 23.5 $\mu$m can be stable over time. Similar studies of temporal droplet size stability were carried out in the CMS6-2 chip (as shown in **Figure 7)** CMS6-3 chip (as shown in **Figure 8).** In some embodiments, because of the smaller channel dimensions in the CMS6-2 chip and the CMS6-3 chip, droplet sizes were lower than in the CMS6-1 chip. For the CMS6-2 chip, the 90% confidence interval of the mean droplet size was (13.8 $\pm$ 0.6) $\mu$m with CV = 2.5%. In some embodiments, in the CMS6-3 chip (**Figure 8**), the 90% confidence interval of the mean droplet size can be (5.2 $\pm$ 0.3) $\mu$m with CV = 3.5%. In some examples, a CV value slightly above 3% can be attributed to larger error in estimating the size of smaller droplets.

Average droplet diameter ($\mu$m) can be calculated every 30 minutes, 120 minutes, 240 minutes, and 360 minutes for every junction (i.e., J1, J2, J3, J4, J5, J6, and J7) on the MC chip. The coefficient of variance can be calculated as follows:

$$CV = \frac{STDEV}{Average}\ x\ 100$$

The particle morphology can be visualized using confocal laser scanning microscopy (CLSM). A suspension of microparticles or dried microparticles can be placed on a microscope slide and excited with argon laser at a wavelength of 488 nm and helium-neon laser with a wavelength of 543 nm. To enhance the observation of polymer-drug distribution, the total emission can be divided into two images that can be captured by two separate photomultiplier tubes (PMTs): PMT1 can capture fluorescence at 515$\pm$30 nm (green region) and PMT2 can capture fluorescence above 570 nm (yellow-red region).

Scanning electron microscopy (SEM) can be utilized to investigate a surface morphology of the particles and the efficiency of the developed washing procedure. In some embodiments, all micrographs can be taken with an aperture size of about 30 $\mu$m using a beam current of about 2.1 nA and an accelerating about voltage of 10 kV. The washing can be considered successful if no PVA crystals could be found on SEM images, as well as no PVA film bridges between any two particles. Particles with smooth clean surface and no sign of particle-particle bridges, can be considered properly washed.

Imaging can be carried out using a focused ion beam-scanning electron microscope (FIB-SEM) which combines ultra-high-resolution SEM and precise FIB etching and deposition. The FIB acceleration voltage can be about 30 kV and an ion beam current can be about 20 nA. In some examples, the cross section can be cleaned at about 7 nA with a final cleaning at about 3nA. In some embodiments, to preserve the exposed cross-section surface during imaging at 2 min/image, the current can be reduced to 30 pA.

X-ray diffractometry (XRD) can be used to analyze patterns of pure SRL, blank PLGA particles, pure PLGA, and SRL-loaded PLGA particles (1:1 SRL:PLGA blend)

Attenuated total reflection-Fourier transform infrared (ATR-FTIR) spectroscopy can be used to ascertain the encapsulation of drugs in the produced microparticles. About 2- about 3 mg of the powdered sample can be placed onto the universal diamond ATR top-plate and ATR-FTIR spectra can be recorded over the about 4000-400 cm$^{-1}$ range. In some samples, the spectrum can be acquired within about 32 s.

Example 3: Drug Loading

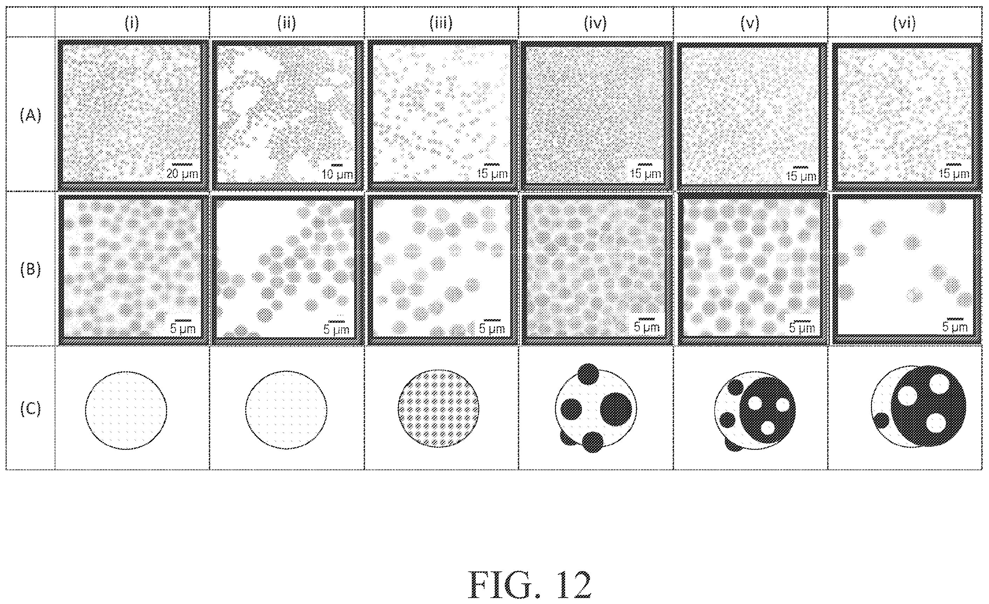

**Figure 12** illustrates the effect of drug loading on the particle morphology for the particle size between 4.1 $\mu$m and 4.5 $\mu$m. As shown, the drug loading was: (i) 33 wt% (S4); (ii) 42 wt% (S5); (iii) 44 wt% (S6); (iv) 45 wt% (S7); (v) 46 wt% (S8); (vi) 50 wt% (S9). The experimental conditions and emulsion formulations are shown in **Table 2.** The images of **Figure 12** are obtained using 20X (A) and 60X (B) magnification lenses. In some embodiments, although both the drug and polymer exhibited fluorescence, Nile red showed a higher adsorption when adsorbed to the drug molecules. As shown in **Figure 12,** drug-polymer distribution within the microparticles can be observed on CLSM images with brighter regions corresponding to SRL and darker areas corresponding to PLGA.

In some examples, drug separation can be dependent on the drug loading. The extent of separation can range from a uniform drug distribution at the drug loading of about 33 wt% and 42 wt%, *(see* (i)(A)-(i)(C) and (ii)(A)-(ii)(C)), to SRL patches embedded within a spherical PLGA matrix at the drug loading of about 44 wt% and 45 wt%, *(see* (iii)(A)-(iii)(C) and (iv)(A)-(iv)(C)), to patchy Janus morphologies at the drug loading of about 46 wt% and 50 wt%, *(see* (v)(A)-(v)(C) and (vi) (A)-(vi)(C)). In some embodiments, the patchy and patchy Janus morphologies are not stable during storage in aqueous phase and slowly evolve into fully separated Janus morphology due to Ostwald ripening. In some examples, drug from small patches can dissolve into the surrounding aqueous phase and get deposited onto larger patches. In some embodiments, this process can also occur at a later stage of the solvent evaporation process through internal diffusion of SRL facilitated by the residual solvent. In some embodiments, the same patch coarsening process can occur in PCL-PLA composite particles leading to various particle morphologies.

X-ray Diffractometry (XRD) analysis was carried out on freeze-dried S4 (see **Table 2**) microparticles to reveal the presence of any impurities in the sample and assess its crystallinity. Encapsulation of SRL within PLGA can be confirmed by XRD analysis. The absence of sharp peaks in the XRD pattern of SRL-loaded PLGA particles indicates that SRL was either molecularly dispersed in PLGA or present in the form of amorphous domains.

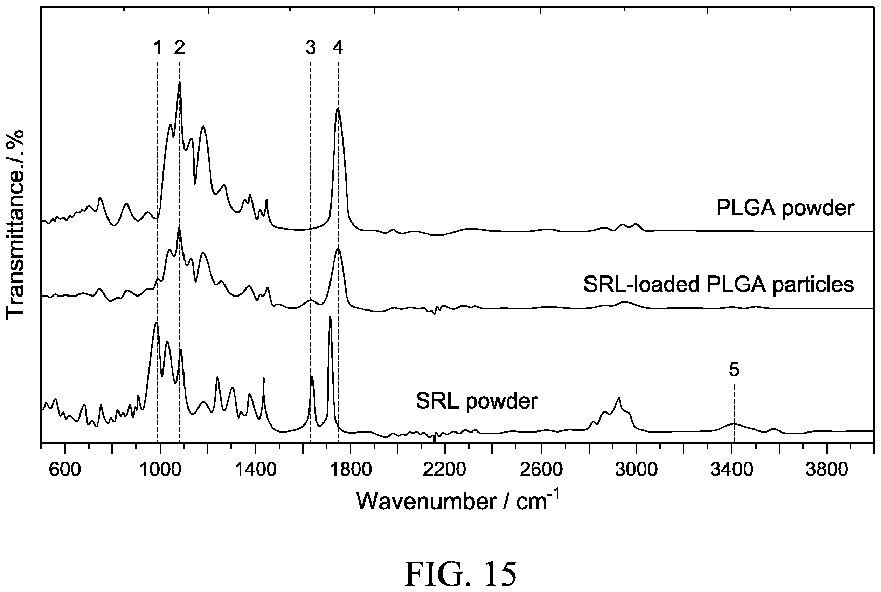

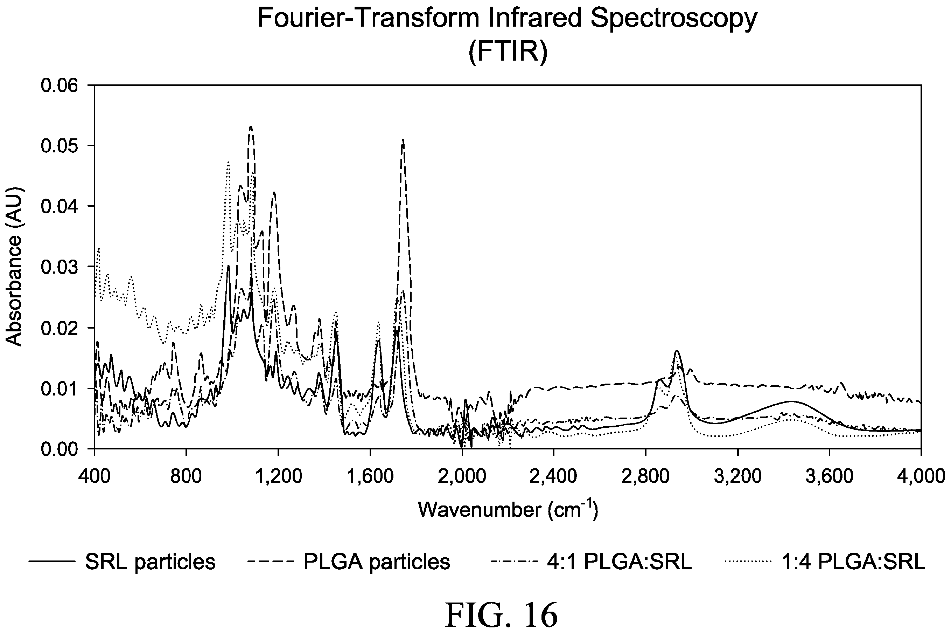

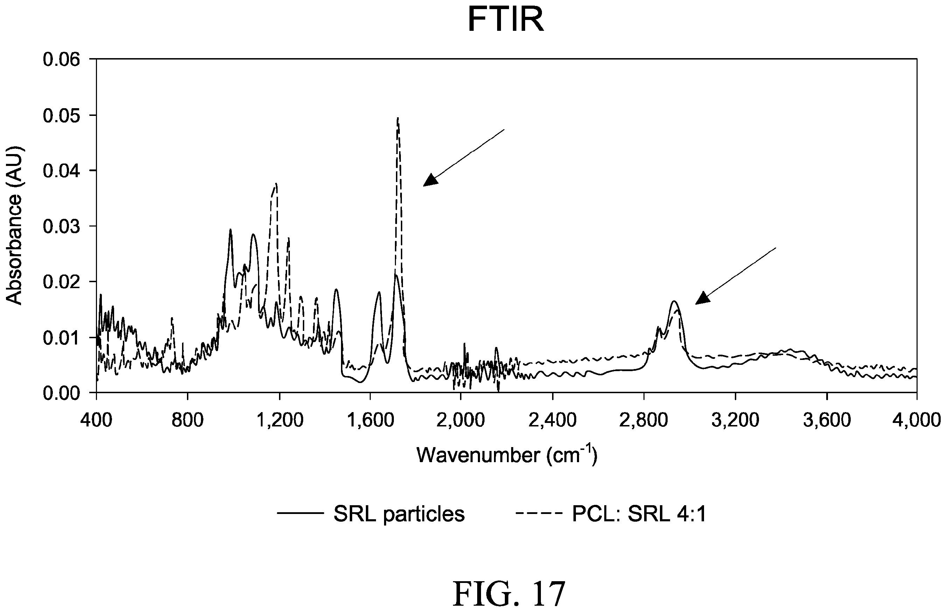

Fourier-Transform Infrared Spectroscopy (FTIR) analysis was carried out on Sample S4 (see **Table 2**) microparticles. **Figure 15** shows the FTIR spectra for pure PLGA, pure drug, and drug loaded PLGA particles. Observed IR absorption peaks reveals the presence of characteristic bonds in PLGA and SRL. For example, the peaks at approximately 1000 cm$^{-1}$ (1) and 1640 cm$^{-1}$ (3) for SRL were due to the out-of-plane C-H bending vibrations in -CH=CH- bonds and the C=C stretching vibrations, respectively. These two bonds do not exist in PLGA and consequently, no peaks were found in the PLGA spectrum at these frequencies. On the other hand, strong peaks at approximately 1100 cm$^{-1}$ (2) and 1750 cm$^{-1}$ (4) for PLGA occurred due to the C-O-C and C=O stretching vibrations in ester groups, respectively. The peak at approximately 3400 cm$^{-1}$ (5) for SRL was due to the O-H stretching vibrations. In PLGA macromolecules, the O-H bonds are present only in terminal groups. No new peaks were found when ATR-FTIR was carried out with drug-encapsulated microparticles as all peaks identified in drug-loaded PLGA particles corresponded to those in the individual components. This indicates no drug-polymer reaction during particle formation. **Figure 15** also shows that SRL was incorporated uniformly within the particles rather than separated onto the surface of the particles since the peaks (1). (3), and (5) are negligible for SRL-loaded PLGA particles. **Figure 16** illustrates a graph showing the FTIR patterns of SRL particles, PLGA particles, particles with a PLGA:SRL ratio of 4:1, and particles with a PLGA:SRL ratio of 1:4. Similar to what was discussed above, **Figure 16** illustrates that with homogenous particles, phase separation does not occur. **Figure 16** demonstrates the successful encapsulation of SRL in PLGA as well that particles with different SRL content can be easily identified. **Figure 17** illustrates another graph showing the FTIR patterns of SRL particles and particles having a PCL:SRL ratio of 4:1.

Example 4: Drug Release

SRL release can occur much faster in dissolution media than in pure PBS. A burst release from PLGA particles can occur within the first 1 hour, but then plateaus for 1 week. In comparison, in dissolution medium, both PLGA and PCL particles showed 68-87% of SRL was released after about 4 hours.

A 100 mL of dissolution medium can be prepared in a volumetric flask by mixing 10 mL of 20 vol% polysorbate 20 (Tween 20), about 10 mL of 200 proof ethanol, about 25.9 mL of 5.8 M N,N-diethylnicotinamide (DENA) solution, about 44.1 mL of Milli-Q water, and about 10 mL of 10× phosphate-buffered saline (PBS) at 37 °C for 1 h. The prepared solution can be stored at about 4°C until use. To measure SRL drug release profile, about 2 mg of freeze-dried microspheres can be placed into a microcentrifuge tube and 2 mL of the dissolution medium can be added. The tube can be capped and alternately sonicated and vortex-agitated for about 2 min to break apart any particle aggregates and fully suspend individual particles in the solution. The tube can then be placed in an agitator rotating at 250 rpm and incubated for about 25 min at 37 °C. The tube can then be removed from the agitator and centrifuged for about 5 min at 13,000 rpm. The supernatant can be extracted from the sample using a syringe and transferred into a labelled HPLC vial. The removed supernatant can be replenished with fresh dissolution medium and the procedure can be repeated in the same way for the samples collected after any one of 1 hour, 2 hours, 3 hours, 4 hours, 5 hours, 7 hours, and 24 hours. After 24 hours, a quenched sample can be prepared by adding about 2 mL of acetonitrile (ACN) to fully dissolve drug-deprived particles and release the remaining drug. In some embodiments, all samples were stored at about -20 °C before analysis. The concentration of SRL in the samples can be determined by High Performance Liquid Chromatography (HPLC).

Example 5: Preparation of SRL Microspheres

Pure sirolimus microspheres were generated using a junction microfluidic chip that includes 3D flow focusing junctions. The dispersed phase consisted of 96.5 wt% DCM in which 3.5 wt% of SIR was dissolved. The continuous phase consisted of 2 wt% PVA in deionized water. Solid particles were obtained from the collected oil-in-water emulsion droplets after solvent evaporation. Briefly, the vial containing the collected droplets was left open in a fume cupboard at room temperature for DCM to evaporate. Indeed, despite a low solubility of DCM in water (17.5 g/L at 25°C), it diffuses out of the droplets into the surrounding aqueous phase. Then, because it is highly volatile (boiling point 39.6°C), DCM evaporates from the air/liquid interface in the vial into the surrounding air. This gradual solvent depletion from the droplets leads to an increase in the polymer and drug concentration which eventually causes the droplets to solidify into particles. The particles were then washed with DI water to remove the PVA from the surface of the particles and frozen in a standard laboratory freezer at -20°C. The majority of volatile compounds (DCM and water) were then removed from the sample at a pressure of around 40 Pa and a condenser temperature of around -50°C for 24 hours. The sample was then reheated at 25°C for 6 hours to remove the remaining amount of solvents.

As discussed with the SRL-PLGA microspheres, the success of droplet generation can depend on successful restoration of a clean hydrophilic surface of the silicon chip after usage (in some embodiments, the chip is reusable). In some embodiments, organic contaminants can be partially removed by ultrasound treatment of the chip by a household detergent solution. In some embodiments, a chemical treatment of the chip may be performed after the ultrasound treatment, which can involve oxygen or ozone plasma oxidation of organic contaminants in a plasma reactor. Hydrophilic chip surfaces can keep a low contact angle between organic droplets and channel walls in the chip. Since the step emulsification process is based on Laplace pressure difference and curvature imbalance along the droplet interface, properly restoration of hydrophilic surfaces can allow uniform droplets to be generated in the chip continuously for at least 24 hours.

In some embodiments, the continuous phase channels of the microfluidic chip can be cleaned by flowing soapy water and warm distilled water through the continuous phase channels. In some examples, the warm distilled water can be flowed over the continuous phase channels for 5 minutes at a high flow rate of between 200 $\mu$L/min - 300 $\mu$L/min. The dispersed phase channels of the microfluidic chip can be cleaned by flowing pure solvent (e.g., DCM) through the dispersed phase channels. In some examples, the pure solvent can be flowed over the dispersed phase channels for 5 minutes at a high flow rate of between 200 $\mu$L/min - 300 $\mu$L/min. In some embodiments, the microfluidic chip can also be deep cleaned with a 0.1 M to 0.5 M sodium hydroxide (NaOH) solution. This deep clean can be done once a week or at least once every two weeks to make sure that it retains its hydrophilic properties.

In some embodiments, the used chips were washed in an ultrasound bath set at 10% power using a mild detergent dissolved in a mixture of water and ethanol. The chip can then be rinsed with Milli-Q water, oven dried, and transferred to a plasma cleaner to oxidize persistent organic contaminants that could not be removed by the detergent. In some examples, the plasma oxidation can be performed at high vacuum of $1\times10^{-5}$ mbar for about 15 min (pure $O_2$) or about 20 min (about 25% O2 and about 75% Ar). The clean microfluidic chip can then be placed on a watch glass in a beaker containing the continuous phase and sonicated for about 20 min. The continuous phase can then be replaced, and the chip stored in the fresh continuous phase. To evaluate the cleaning process, the contact angle between a water droplet and a dry chip surface was measured. In some embodiments, the contact angle of a water drop with a contaminated silicon surface can be approximately 60°. After washing with dichloromethane (DCM), the contact angle was found to be reduced to 32.4° as a result of the partial removal of organic contaminants. After the microfluidic chip was treated by plasma oxidation, the contact angle was further reduced to 16.9° as a result of the complete removal of persistent organic layer.

In some embodiments, surface treatment using the previously discussed procedure can lead to sustained droplet generation for at least 24 hours for all runs. In some examples, any wetting of the terrace walls by the dispersed phase can reduce Laplace pressure on the terrace ($p_1$), as a result of flattening the dispersed phase interface in the orthogonal direction, the radius of curvature can become greater than $D_{MC}$ and it approaches infinity for the wetting angle 90°. In some embodiments, this can lead to reduced driving force for droplet pinch-off and may cause a continuous outflow of the dispersed phase from the microchannels and formation of large polydisperse droplets. In some embodiments, although the terrace wall wettability was slightly altered after 24 hours compared to the case after one hour due to prolonged contact of silicon surface with emulsion ingredients (DCM, SRL and PVA), no change in droplet size was observed.

In some examples, after the microspheres are produced by step microfluidic emulsification, the solvent is removed from the microsphere into the surrounding aqueous phase.

In some embodiments, washing the microspheres to remove PVA can affect the particle interactions with cell membranes in the body by increasing the hydrophilicity of the surface. The amount of residual PVA adsorbed on the particle surface can depend on the miscibility of the solvent blend with water. A higher amount of adsorbed PVA can be expected in the particles prepared from the solvent blend with a higher amount of IPAc. In some examples, this can be due to higher aqueous solubility of isopropyl acetate (IPAc) compared to DCM. In some embodiments, when droplets are formed in a PVA solution, the PVA can be absorbed on to the droplet surfaces in such a way that hydrophobic acetate tails of PVA molecules can be immersed in the organic phase, while hydrophilic alcohol groups will be exposed to the aqueous phase. In some embodiments, after solvent evaporation, the PVA coating can remain on particle surfaces and enhanced hydrophilic properties of the particles because of exposed -OH groups on the surface. In some embodiments the increased absorption of residual PVA can result in increased hydrophilicity of the particles.

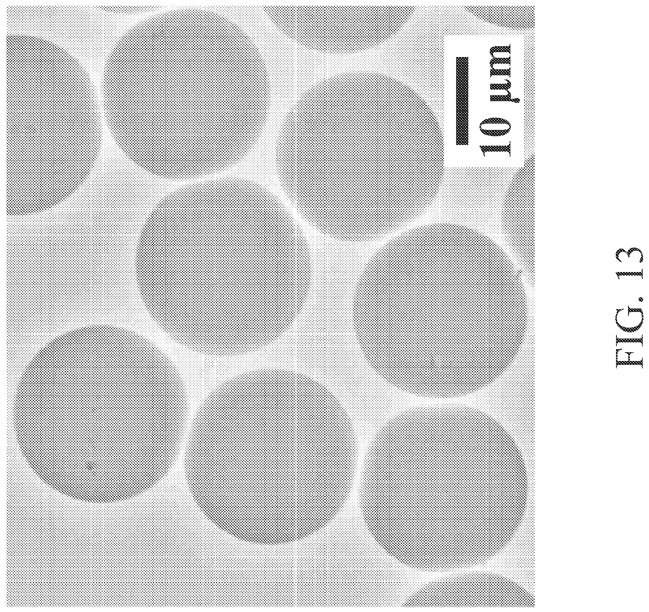

**Figure 13** illustrates an SEM image of microparticles with a PLGA:SRL ratio of 0:1. As shown, when the microparticles are 100% SRL, the formed microparticles are homogenous and fully amorphous due to their regular spherical shape. If the particles have a crystalline structure they would have non-spherical shape with sharp edges due to a regular arrangement of drug molecules in the crystal lattice.

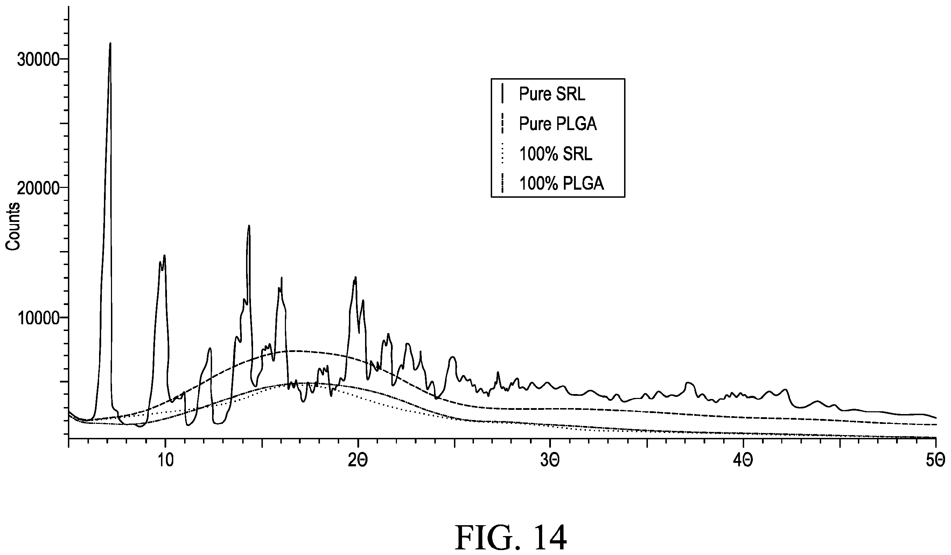

In some embodiments, X-ray Diffractometry (XRD) analysis can be carried out on freeze-dried microparticles to reveal the presence of any impurities in the sample and to assess its crystallinity. **Figure 14** illustrates a graph comparing the XRD patterns of pure SRL, pure PLGA, 100% SRL particles, and 100% PLGA particles. As shown, pure SRL showed about 50%- about 60% crystallinity, pure PLGA showed 0%- about 2% crystallinity, 100% SRL particles showed about 1%- about 2% crystallinity, and 100% PLGA particles showed 0%- about 2% crystallinity. Sharp peaks at 2θ 6.8°, 9.8°, 12.2°, 14.5°, 15.9°, 19.9°, 20.2°, 21.5°, 22.5°, 23.3° and 25° in the XRD spectrum of raw SRL powder indicate that SIR was in a crystalline form and the degree of crystallinity of this sample was estimated to be 56%. These sharp peaks, however, were not observed in the XRD spectrum of the pure SRL particles, suggesting their amorphous structure. These results imply that the particle fabrication process induced a structural change in SRL from crystalline to amorphous. Fourier-Transform Infrared Spectroscopy (FTIR) analysis was carried out on Sample S4 (see **Table 2**) microparticles. **Figure 16** illustrates a graph showing the FTIR patterns of SRL particles, PLGA particles, particles with a PLGA:SRL ratio of 4:1, and particles with a PLGA:SRL ratio of 1:4. **Figure 16** demonstrates the successful encapsulation of SRL in PLGA as well that particles with different SRL content can be easily identified. **Figure 17** illustrates the FTIR patterns of pure SRL particles and particles having a PCL:SRL ratio of 4:1.

**Claims**

1. A method of generating agglomeration-resistant monodispersed and homogenous sirolimus microspheres, the method comprising:

   producing a droplet comprising sirolimus using microfluidic step emulsification, wherein the microfluidic step emulsification comprises injecting a dispersed phase composition comprising sirolimus and a solvent into a continuous phase composition;
   removing solvent from the droplet to form sirolimus microspheres that are free of polymer; and
   washing the sirolimus microspheres to remove the continuous phase composition.

2. The method of Claim 1, further comprising freeze drying the sirolimus microspheres to remove solvent from the sirolimus microspheres.

3. The method of Claim 2, wherein freeze drying the sirolimus microspheres is configured to reduce particle size by 10-15%.

4. The method of any one of Claims 1 to 3, wherein the sirolimus microspheres are formed using a dripping regime.

5. The method of any one of Claims 1 to 4, wherein the microfluidic step emulsification uses a microfluidic chip comprising a plurality of junctions; preferably wherein the microfluidic chip includes seven junctions.

6. The method of Claim 5, wherein the microfluidic chip comprises a plurality of 3D flow junctions; preferably wherein the microfluidic chip comprises seven 3D flow junctions.

7. The method of Claim 5 or 6, wherein the microfluidic chip comprises cross-flow channels configured to deliver the dispersed phase composition and parallel arrays of terraced microgrooves configured to deliver the continuous phase composition.

8. The method of Claim 7, wherein the microfluidic chip comprises 10 parallel arrays of terraced microgrooves, wherein the terraced microgrooves have a depth of between about 2 and about 5 μm.

9. The method of any one of Claims 1 to 8, wherein the sirolimus microspheres formed have a diameter between about 1.8 and about 8.4 μm.

10. The method of any one of Claims 1 to 9, wherein the sirolimus microspheres are washed between 6-7 times or wherein the sirolimus microspheres are washed at least 8 times.

11. The method of any one of Claims 1 to 10, wherein washing the sirolimus microspheres further comprises centrifuging a suspension comprising sirolimus microspheres and/or adding an equal volume of 0.05 wt% Tween 20 aqueous solution and vortexing for 5 s to 10 s.

12. The method of any one of Claims 1 to 11, wherein the continuous phase composition does not include isopropyl acetate (IPAc).

13. The method of any one of Claims 1 to 12, wherein the solvent of the dispersed phase composition can include any one of dichloromethane (DCM), acetone, chloroform, methanol, ethanol, ethyl acetate, acetonitrile, and isopropyl acetate.

14. The method of Claim 13, wherein the solvent comprises dichloromethane (DCM).

15. The method of any one of Claims 1 to 14, wherein the continuous phase composition comprises deionized water and a surfactant; preferably wherein the continuous phase composition comprises polyvinyl alcohol (PVA), Tween 80, Triton X-100, sodium dodecyl sulfate (SDS), Pluronic surfactants, for example Pluronic F68 or Pluronic F127, and monomethoxypolyethylene oxide (MPEO)-b-PLA diblock copolymers.

**Patentansprüche**

1. Verfahren zur Erzeugung agglomerationsbeständiger, monodispergierter und homogener Sirolimus-Mikrosphären, wobei das Verfahren umfasst:

   Herstellen eines Sirolimus umfassenden Tröpfchens unter Verwendung mikrofluidischer Stufenemulgierung, wobei die mikrofluidische Stufenemulgierung das Injizieren einer Zusammensetzung einer dispergierten Phase, umfassend Sirolimus und ein Lösungsmittel, in eine Zusammensetzung einer kontinuierlichen Phase umfasst; Entfernen von Lösungsmittel aus dem Tröpfchen, um Sirolimus-Mikrosphären zu bilden, die frei von Polymer sind; und
   Waschen der Sirolimus-Mikrosphären, um die Zusammensetzung der kontinuierlichen Phase zu entfernen.

2. Verfahren nach Anspruch 1, ferner umfassend Gefriertrocknen der Sirolimus-Mikrosphären, um Lösungsmittel aus den Sirolimus-Mikrosphären zu entfernen.

3. Verfahren nach Anspruch 2, wobei das Gefriertrocknen der Sirolimus-Mikrosphären dazu ausgestaltet ist, die Partikelgröße um 10-15 % zu reduzieren.

4. Verfahren nach einem der Ansprüche 1 bis 3, wobei die Sirolimus-Mikrosphären unter Verwendung eines Tropfregimes gebildet werden.

5. Verfahren nach einem der Ansprüche 1 bis 4, wobei die mikrofluidische Stufenemulgierung einen mikrofluidischen Chip verwendet, der eine Vielzahl von Übergängen umfasst; vorzugsweise wobei der mikrofluidische Chip sieben Übergänge einschließt.

6. Verfahren nach Anspruch 5, wobei der mikrofluidische Chip eine Vielzahl von 3D-Strömungsübergängen umfasst; vorzugsweise wobei der mikrofluidische Chip sieben 3D-Strömungsübergänge umfasst.

7. Verfahren nach Anspruch 5 oder 6, wobei der mikrofluidische Chip Kreuzströmungskanäle, die dazu ausgestaltet sind, die Zusammensetzung der dispergierten Phase zuzuführen, und parallele Anordnungen von terrassierten Mikrorillen umfasst, die dazu ausgestaltet sind, die Zusammensetzung der kontinuierlichen Phase zuzuführen.

8. Verfahren nach Anspruch 7, wobei der mikrofluidische Chip 10 parallele Anordnungen von terrassierten Mikrorillen umfasst, wobei die terrassierten Mikrorillen eine Tiefe zwischen etwa 2 und etwa 5 $\mu$m aufweisen.

9. Verfahren nach einem der Ansprüche 1 bis 8, wobei die gebildeten Sirolimus-Mikrosphären einen Durchmesser zwischen etwa 1,8 und etwa 8,4 $\mu$m aufweisen.

10. Verfahren nach einem der Ansprüche 1 bis 9, wobei die Sirolimus-Mikrosphären zwischen 6-7 Mal gewaschen werden oder wobei die Sirolimus-Mikrosphären mindestens 8 Mal gewaschen werden.

11. Verfahren nach einem der Ansprüche 1 bis 10, wobei das Waschen der Sirolimus-Mikrosphären ferner das Zentrifugieren einer Suspension, umfassend Sirolimus-Mikrosphären, und/oder das Zugeben eines gleichen Volumens einer wässrigen 0,05 wt% Tween-20-Lösung und Vortexen für 5 s bis 10 s umfasst.

12. Verfahren nach einem der Ansprüche 1 bis 11, wobei die Zusammensetzung der kontinuierlichen Phase kein Isopropylacetat (IPAc) einschließt.

13. Verfahren nach einem der Ansprüche 1 bis 12, wobei das Lösungsmittel der Zusammensetzung der dispergierten Phase ein beliebiges von Dichlormethan (DCM), Aceton, Chloroform, Methanol, Ethanol, Ethylacetat, Acetonitril und Isopropylacetat einschließen kann.

14. Verfahren nach Anspruch 13, wobei das Lösungsmittel Dichlormethan (DCM) umfasst.

15. Verfahren nach einem der Ansprüche 1 bis 14, wobei die Zusammensetzung der kontinuierlichen Phase deionisiertes Wasser und ein Tensid umfasst; vorzugsweise wobei die Zusammensetzung der kontinuierlichen Phase Polyvinylalkohol (PVA), Tween 80, Triton X-100, Natriumdodecylsulfat (SDS), Pluronic-Tenside, zum Beispiel Pluronic F68 oder Pluronic F127, und Monomethoxypolyethylenoxid (MPEO)-b-PLA-Diblockcopolymere umfasst.


**Revendications**

1. Procédé de génération de microsphères de sirolimus monodispersées et homogènes résistantes à l'agglomération, le procédé comprenant :

   la production d'une gouttelette comprenant du sirolimus à l'aide d'une émulsification microfluidique par étape, dans lequel l'émulsification microfluidique par étape comprend l'injection d'une composition de phase dispersée comprenant du sirolimus et un solvant dans une composition de phase continue ;
   le retrait du solvant de la gouttelette pour former des microsphères de sirolimus qui sont exemptes de polymère ; et
   le lavage des microsphères de sirolimus pour retirer la composition de phase continue.

2. Procédé selon la revendication 1, comprenant en outre la lyophilisation des microsphères de sirolimus pour retirer le solvant des microsphères de sirolimus.

3. Procédé selon la revendication 2, dans lequel la lyophilisation des microsphères de sirolimus est configurée pour réduire la taille des particules de 10-15 %.

4. Procédé selon l'une quelconque des revendications 1 à 3, dans lequel les microsphères de sirolimus sont formées à l'aide d'un régime d'égouttement.

5. Procédé selon l'une quelconque des revendications 1 à 4, dans lequel l'émulsification microfluidique par étape utilise une puce microfluidique comprenant une pluralité de jonctions ; de préférence dans lequel la puce microfluidique inclut sept jonctions.

6. Procédé selon la revendication 5, dans lequel la puce microfluidique comprend une pluralité de jonctions d'écoulement 3D ; de préférence dans lequel la puce microfluidique comprend sept jonctions d'écoulement 3D.

7. Procédé selon la revendication 5 ou 6, dans lequel la puce microfluidique comprend des canaux à écoulement transversal configurés pour distribuer la composition de phase dispersée et des réseaux parallèles de micro-rainures en terrasses configurés pour distribuer la composition de phase continue.

8. Procédé selon la revendication 7, dans lequel la puce microfluidique comprend 10 réseaux parallèles de micro-rainures en terrasses, dans lequel les micro-rainures en terrasses ont une profondeur comprise entre environ 2 et environ 5 $\mu$m.

**9.** Procédé selon l'une quelconque des revendications 1 à 8, dans lequel les microsphères de sirolimus formées ont un diamètre compris entre environ 1,8 et environ 8,4 μm.

**10.** Procédé selon l'une quelconque des revendications 1 à 9, dans lequel les microsphères de sirolimus sont lavées entre 6-7 fois ou dans lequel les microsphères de sirolimus sont lavées au moins 8 fois.

**11.** Procédé selon l'une quelconque des revendications 1 à 10, dans lequel le lavage des microsphères de sirolimus comprend en outre la centrifugation d'une suspension comprenant des microsphères de sirolimus et/ou l'ajout d'un volume égal de solution aqueuse de Tween 20 à 0,05 wt% en poids et une agitation au vortex pendant 5 s à 10 s.

**12.** Procédé selon l'une quelconque des revendications 1 à 11, dans lequel la composition de phase continue n'inclut pas d'acétate d'isopropyle (IPAc).

**13.** Procédé selon l'une quelconque des revendications 1 à 12, dans lequel le solvant de la composition de phase dispersée peut inclure l'un quelconque parmi le dichlorométhane (DCM), l'acétone, le chloroforme, le méthanol, l'éthanol, l'acétate d'éthyle, l'acétonitrile, et l'acétate d'isopropyle.

**14.** Procédé selon la revendication 13, dans lequel le solvant comprend du dichlorométhane (DCM).

**15.** Procédé selon l'une quelconque des revendications 1 à 14, dans lequel la composition de phase continue comprend de l'eau désionisée et un tensioactif ; de préférence dans lequel la composition de phase continue comprend de l'alcool polyvinylique (PVA), du Tween 80, du Triton X-100, du dodécylsulfate de sodium (SDS), des tensioactifs Pluronic, par exemple du Pluronic F68 ou du Pluronic F127, et des copolymères diblocs monométhoxypolyéthylène oxyde (MPEO)-b-PLA.

FIG. 1A

EP 4 489 753 B1

FIG. 1B

FIG. 1C

FIG. 1D

FIG. 1E

EP 4 489 753 B1

FIG. 2

FIG. 3A

FIG. 3B

FIG. 3C

FIG. 4A

FIG. 4B

FIG. 4C

FIG. 4D

Scale bar is 100 μm

FIG. 5

FIG. 6

EP 4 489 753 B1

FIG. 7

FIG. 8

FIG. 9A

FIG. 9C

FIG. 9B

FIG. 10A      FIG. 10B      FIG. 10C      FIG. 10D

FIG. 11A     FIG. 11B     FIG. 11C     FIG. 11D

FIG. 12

EP 4 489 753 B1

FIG. 13

FIG. 14

FIG. 15

FIG. 16

# FTIR

FIG. 17

EP 4 489 753 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Microfluidic preparation of PLGA microspheres as cell carriers with sustainable Rapa release. **ZHU CHENGCHENG et al.** JOURNAL OF BIOMATERIALS SCIENCE. POLYMER EDITION, 19 April 2019, vol. 30, 737-755